# EUROPEAN PATENT APPLICATION

(11) **EP 3 929 213 A1**
(43) Date of publication of application: **29.12.2021**
(21) Application number: 20758681.9
(22) Date of filing: 20.02.2020
(51) Int. Cl.: C07K 16/28, C07K 16/46, C12N 15/13, C12N 15/63, C12N 5/20, A61K 39/395, A61P 35/00

(54) **ANTI-PD-L1 ANTIBODY AND USE THEREOF**

(30) Priority: 21.02.2019 WO PCT/CN2019/075654
(71) Applicant: Eucure (Beijing) Biopharma Co., Ltd, Beijing 100176 (CN)
(72) Inventor: YANG, Yi, Beijing 100176 (CN); XIE, Jingshu, Beijing 100176 (CN); DONG, Chunyan, Beijing 100176 (CN); YANG, Fang, Beijing 100176 (CN); LU, Chengyuan, Beijing 100176 (CN); SHEN, Yuelei, Beijing 100176 (CN); NI, Jian, Beijing 100176 (CN); GUO, Ya'nan, Beijing 100176 (CN); CHEN, Yunyun, Beijing 100176 (CN)
(74) Representative: Fish & Richardson P.C.
(86) International application number: PCT/CN2020/075983
(87) International publication number: WO 2020/169062

(57) **Abstract**

The present application provides an anti-PD-L1 antibody, an antigen-binding fragment thereof, and use thereof. The present application also provides a multispecific antibody such as a bispecific antibody, a conjugate, and a composition comprising the anti-PD-L1 antibody or the antigen-binding fragment thereof, and use thereof in treatment of diseases such as cancer..

## Description

### Technical Field

The present invention relates to the field of biomedicine. More specifically, the present invention relates to an anti-PD-L1 antibody and use thereof, especially in treatment of cancer.

### Background Art

Cancer is currently one of the diseases leading to the highest mortality in humans. According to statistics from the World Health Organization, in 2012 the number of malignant tumor incidence and death cases worldwide reached 14 million and 8.2 million, respectively. There were 3.07 million newly diagnosed cancer cases and 2.2 million deaths in China. In recent years, immune checkpoints have been considered as effective potential targets for the treatment of various cancers, and the development of antibody drugs against immune checkpoints has attracted more and more attention.

The full names of PD-1 and PD-L1 are programmed cell death-1 and programmed cell death-ligand 1, respectively, which are mainly involved in the body's immune regulation process such as autoimmunity and tumor immunity as important members of the immunoglobulin superfamily of costimulatory molecules. PD-1, type I transmembrane protein with a size of 40 kDa, is an inhibitory receptor mainly expressed on activated T cells. When combined with its ligand PD-L1, it can significantly inhibit the activation and proliferation of T cells. There are currently two known PD-1 ligands, namely PD-L1 (also known as B7-H1) and PD-L2 (also known as B7-DC), respectively. The human PD-L1 gene is located on chromosome 9p24, and encodes a type I transmembrane protein of 290 amino acids. It is composed of extracellular IgV and IgC domains, a hydrophobic transmembrane domain and an intracellular domain of 30 amino acids. PD-L1 is widely expressed on the surface of a variety of immune cells, epithelial cells and tumor cells. PD-L2 is mainly expressed on the surface of immune cells. Current research has found that tumor cells can inhibit the activation and proliferation of tumor antigen-specific T cells by highly expressing PD-L1 molecules to bind to the receptor PD-1 on the surface of T cells, and transmitting negative regulatory signals, thereby evading the body's immune monitoring and killing.

In recent years, monoclonal antibodies targeting PD-1/PD-L1 proteins have been used to block the binding of PD-1/PD-L1 to promote the activation and proliferation of T cells in the body to achieve the purpose of killing tumor cells. They have been applied to a variety of tumors, such as melanoma, lymphoma, bladder cancer, non-small cell lung cancer, head and neck cancer, and colon cancer, and have achieved certain therapeutic effects. Tumor immunotherapy has become one of the research hotspots and development directions for new drug worldwide.

At present, 3 anti-PD-L1 antibodies have been approved by the FDA, namely the anti-PD-L1 antibody Atezolizumab developed by Roche (for bladder cancer, locally advanced or metastatic urothelial carcinoma, metastatic non-small cell lung cancer), the anti-PD-L1 monoclonal antibody Avelumab jointly developed by Merck/Pfizer (for rare skin cancer, Merkel cell carcinoma, and the like), and Durvalumab developed by AstraZeneca (for advanced or metastatic urothelial carcinoma). In addition to single drugs, anti-PD-L1 antibodies are also being tried in combination. For example, Durvalumab is currently used in combination with 13 drugs with different mechanisms, including OX40, MEK, CTLA4, and the like. Monoclonal antibodies targeting PD-L1 that have been on the market have their own advantages and disadvantages in the treatment of cancer, but they are expensive and cannot be widely used and popularized. Therefore, the development of antibody drugs that have a wider application range and can block the PD-1/PD-L1 signaling pathway more efficiently will provide the possibility for the treatment of a variety of tumors and immune system-related diseases, and has huge application potential and market value.

### Summary of the Invention

Unless otherwise defined herein, the scientific and technical terms and abbreviations thereof used in conjunction with the present invention shall have the meanings commonly understood by one of ordinary skill in the art to which the present invention belongs. Some of the terms and abbreviations used herein are listed as below.
Antibody, Ab;
Immunoglobulin, Ig;
Heavy chain, HC;
Light chain, LC;
Heavy chain variable domain, VH;
Heavy chain constant domain, CH;
Light chain variable domain, VL;
Light chain constant domain, CL;
Complementarity determining region, CDR refers to the antigen complementary binding region of an antibody;
Fab fragment: antigen binding fragment, Fab;
Fc fragment: fragment crystallizable region, Fc;
Monoclonal antibodies, mAbs;
Antibody-dependent cell-mediated cytotoxicity, ADCC;
Complement dependent cytotoxicity, CDC;
Bispecific antibody, BsAb.

As used herein, the term "antibody" refers to an immunoglobulin molecule comprising at least one antigen recognition site and can specifically bind to an antigen. The term "antibody" mentioned in the present invention is understood in its broadest meaning, and comprises a monoclonal antibody, a polyclonal antibody, an antibody fragment, a multispecific antibody comprising at least two different antigen binding domains (such as bispecific antibodies). The antibody also includes a murine antibody, a chimeric antibody, a humanized antibody, a human antibody, and an antibody from other sources. The antibody of the present invention can be derived from any animal, including but not limited to an immunoglobulin molecule of a human, a non-human primate, a mouse or a rat. The antibody may contain additional changes, such as an unnatural amino acid, a mutation in Fc effector function, and a mutation in a glycosylation site. The antibody also includes a post-translationally modified antibody, a fusion protein comprising the antigenic determinant of the antibody, and an immunoglobulin molecule comprising any other modifications to the antigen recognition site, as long as these antibodies exhibit the desired biological activity.

According to the amino acid sequence of the heavy chain constant region of the antibody, the immunoglobulin can be divided into five categories: IgA, IgD, IgE, IgG, and IgM. They can also be further divided into different subclasses (isotypes), such as IgG1, IgG2, IgG3, IgG4, IgA1 and IgA2. According to the amino acid sequence of the light chain, the light chain can be classified as a lambda chain or a kappa chain. The antibody of the present invention can be of any type (such as IgA, IgD, IgE, IgG, and IgM) or subclass (such as IgG1, IgG2, IgG3, IgG4, IgA1 or IgA2).

The term "antigen binding fragment" includes but is not limited to: a Fab fragment having VL, CL, VH and CH1 domains; a Fab' fragment, which is a Fab fragment with one or more cysteine residues at the C-terminus of the CH1 domain; a Fd fragment having VH and CH1 domains; a Fd' fragment having VH and CH1 domains and one or more cysteine residues at the C-terminus of the CH1 domain; a Fv fragment having VL and VH domains of the single arm of the antibody; a dAb fragment consisting of VH domain or VL domain; an isolated CDR region; a F(ab')₂ fragment, which is a bivalent fragment comprising two Fab' fragments connected by a disulfide bridge at the hinge region; a single-chain antibody molecule (such as single-chain Fv; scFv); a "diabody" with two antigen binding sites, comprising the heavy chain variable domain (VH) connected to the light chain variable domain (VL) in the same polypeptide chain; a "linear antibody" comprising a pair of tandem Fd segments (VH-CH1-VH-CH1), which form a pair of antigen binding regions together with the complementary light chain polypeptide; and a modified form of any of the foregoing substances, which retains antigen binding activity.

As used herein, the term "CDR" refers to a complementarity determining region within the variable sequence of an antibody. For each variable region, there are three CDRs in each variable region of the heavy chain and light chain, which are called CDR1, CDR2, and CDR3. The exact boundaries of these CDRs are defined differently according to different systems. The system described by Kabat et al. (Kabat et al, Sequences of Proteins of Immunological Interest (National Institutes of Health, Bethesda, Md. (1987) and (1991)) not only provides a clear residue numbering system suitable for the antibody variable region, but also provides a residue boundary defining three CDRs. These CDRs can be called Kabat CDRs. Each complementarity determining region may comprise amino acid residues from the "complementarity determining region" as defined by Kabat. Chothia et al. (Chothia & Lesk, J. Mol. Biol, 196: 901-917 (1987) and Chothia et al., Nature 342: 877-883 (-1989)) found that some sub-parts in Kabat CDR adopt almost the same peptide skeleton conformation, despite the large diversity at the amino acid sequence level. These sub-parts are referred to as L1, L2, and L3, or H1, H2, and H3, respectively, where "L" and "H" represent light chain and heavy chain regions, respectively. These regions can be referred to as Chothia CDRs, which have boundaries that overlap with Kabat CDRs. There are other CDR boundary definitions that may not strictly follow one of the above systems, but will still overlap with Kabat CDR. The method used herein can utilize CDRs defined according to any of these systems, although the preferred embodiment uses CDRs defined by Kabat or Chothia. As used herein, "antibody variable region" refers to a moiety of the light chain and heavy chain of the antibody molecule comprising the amino acid sequences of the complementarity determining regions (CDRs, namely CDR1, CDR2, and CDR3) and framework regions (FRs). VH refers to the variable domain of the heavy chain. VL refers to the variable domain of the light chain.

The term "chimeric antibody" as used herein refers to an antibody in which the variable region is derived from a non-human species (e.g., derived from a rodent) and the constant region is derived from a different species (e.g., human). The chimeric antibody can be generated by antibody engineering. "Antibody engineering" is a term generally used for different kinds of modification of antibodies, and methods for antibody engineering are well known to those skilled in the art. Therefore, the chimeric antibody can be a genetic or engineered recombinant antibody. Methods of generating chimeric antibodies are known to those skilled in the art, and therefore, the generation of chimeric antibodies can be performed by methods other than those described herein. Chimeric monoclonal antibodies are developed for human therapeutic applications to reduce the expected antibody immunogenicity of non-human antibodies, such as rodent antibodies. They may generally contain non-human (e.g., murine or rabbit) variable regions which are specific for the antigen of interest, and human constant antibody heavy and light chain domain. The term "variable region" or "variable domain" as used in the context of a chimeric antibody refers to a region comprising the CDRs and framework regions of both the heavy and light chains of an immunoglobulin, as described below.

The term "humanized antibody" as used herein refers to a genetically engineered non-human antibody comprising modified human antibody constant domain and non-human variable domain to contain a high level of sequence homology with human variable domains. This can be achieved by grafting six non-human antibody CDRs (they form an antigen binding site together) onto the homologous human acceptor framework region (FR). In order to completely reconstruct the binding affinity and specificity of the parent antibody, it may be necessary to replace the framework residues from the parent antibody (i.e., non-human antibody) into the human framework region (back mutation). Therefore, the humanized antibody may comprise non-human CDR sequences, mainly human framework regions, which optionally comprises one or more amino acid back mutations to the non-human amino acid sequence, and fully human constant regions. Optionally, additional amino acid modifications (which are not necessarily back mutations) can be applied to obtain humanized antibodies with preferred characteristics, such as affinity and biochemical properties and/or additional amino acid mutations can be introduced in the constant region.

As used herein, the term "monoclonal antibody" refers to an antibody obtained from a substantially homogeneous antibody population, that is, each antibody constituting the population is the same, except for possible naturally occurring mutations that may exist in small amounts. Monoclonal antibodies are highly specific and are directed against a single antigen. Furthermore, in contrast to polyclonal antibody preparations usually including different antibodies directed against different determinants (epitopes), each antibody in a monoclonal preparation is directed against the same single determinant on the antigen. As used herein, the term "monoclonal antibody" is not limited to antibodies produced by hybridoma technology, and the modifier "monoclonal antibody" should not be interpreted as requiring the production of antibodies by any specific method.

In the present invention, the inventors generated a new anti-PD-L1 antibody and carried out humanization on it. The aforementioned antibodies and humanized forms thereof can effectively block the binding of PD-1/PD-L1, and show significant therapeutic effects in cancer animal models.

Accordingly, in one aspect, the present invention relates to an anti-PD-L1 antibody or antigen binding fragment thereof, wherein the anti-PD-L1 antibody comprises a heavy chain complementarity determining region (CDR H) comprising:
CDR H1 comprising an amino acid sequence selected from SEQ ID NO: 7, SEQ ID NO: 13, SEQ ID NO: 19, SEQ ID NO: 64, SEQ ID NO: 70, SEQ ID NO: 76 or SEQ ID NO: 82;
CDR H2 comprising an amino acid sequence selected from SEQ ID NO: 8, SEQ ID NO: 14, SEQ ID NO: 20, SEQ ID NO: 65, SEQ ID NO: 71, SEQ ID NO: 77 or SEQ ID NO: 83; and
CDR H3 comprising an amino acid sequence selected from SEQ ID NO: 9, SEQ ID NO: 15, SEQ ID NO: 21, SEQ ID NO: 66, SEQ ID NO: 72, SEQ ID NO: 78 or SEQ ID NO: 84.

In some embodiments, the anti-PD-L1 antibody comprises the CDR H comprising:
a) CDR H1, CDR H2 and CDR H3 comprising SEQ ID NO: 7, SEQ ID NO: 8 and SEQ ID NO: 9, respectively;
b) CDR HI, CDR H2 and CDR H3 comprising SEQ ID NO: 13, SEQ ID NO: 14 and SEQ ID NO: 15, respectively;
c) CDR HI, CDR H2 and CDR H3 comprising SEQ ID NO: 19, SEQ ID NO: 20 and SEQ ID NO: 21, respectively;
d) CDR HI, CDR H2 and CDR H3 comprising SEQ ID NO: 64, SEQ ID NO: 65 and SEQ ID NO: 66, respectively;
e) CDR HI, CDR H2 and CDR H3 comprising SEQ ID NO: 70, SEQ ID NO: 71 and SEQ ID NO: 72, respectively;
f) CDR HI, CDR H2 and CDR H3 comprising SEQ ID NO: 76, SEQ ID NO: 77 and SEQ ID NO: 78, respectively; or
g) CDR HI, CDR H2 and CDR H3 comprising SEQ ID NO: 82, SEQ ID NO: 83 and SEQ ID NO: 84, respectively.

In some embodiments, the anti-PD-L1 antibody also comprises the light chain complementarity determining regions (CDR L) comprising:
CDR L1 comprising an amino acid sequence selected from SEQ ID NO: 10, SEQ ID NO: 16, SEQ ID NO: 22, SEQ ID NO: 67, SEQ ID NO: 73, SEQ ID NO: 79 or SEQ ID NO: 85;
CDR L2 comprising an amino acid sequence selected from SEQ ID NO: 11, SEQ ID NO: 17, SEQ ID NO: 23, SEQ ID NO: 68, SEQ ID NO: 74, SEQ ID NO: 80 or SEQ ID NO: 86; and
CDR L3 comprising an amino acid sequence selected from SEQ ID NO: 12, SEQ ID NO: 18, SEQ ID NO: 24, SEQ ID NO: 69, SEQ ID NO: 75, SEQ ID NO: 81 or SEQ ID NO: 87.

In some embodiments, the anti-PD-L1 antibody comprises the CDR L comprising:
a) CDR L1, CDR L2 and CDR L3 comprising SEQ ID NO: 10, SEQ ID NO: 11 and SEQ ID NO: 12, respectively;
b) CDR L1, CDR L2 and CDR L3 comprising SEQ ID NO: 16, SEQ ID NO: 17 and SEQ ID NO: 18, respectively;
c) CDR L1, CDR L2 and CDR L3 comprising SEQ ID NO: 22, SEQ ID NO: 23 and SEQ ID NO: 24, respectively;
d) CDR L1, CDR L2 and CDR L3 comprising SEQ ID NO: 67, SEQ ID NO: 68 and SEQ ID NO: 69, respectively;
e) CDR L1, CDR L2 and CDR L3 comprising SEQ ID NO: 73, SEQ ID NO: 74 and SEQ ID NO: 75, respectively;
f) CDR L1, CDR L2 and CDR L3 comprising SEQ ID NO: 79, SEQ ID NO: 80 and SEQ ID NO: 81, respectively; or
g) CDR L1, CDR L2 and CDR L3 comprising SEQ ID NO: 85, SEQ ID NO: 86 and SEQ ID NO: 87, respectively.

In a preferred embodiment, the anti-PD-L1 antibody comprises the CDR H and CDR L comprising:
a) CDR H1, CDR H2 and CDR H3 comprising SEQ ID NO: 7, SEQ ID NO: 8 and SEQ ID NO: 9, respectively, and CDR L1, CDR L2 and CDR L3 comprising SEQ ID NO: 10, SEQ ID NO: 11 and SEQ ID NO: 12, respectively;
b) CDR HI, CDR H2 and CDR H3 comprising SEQ ID NO: 13, SEQ ID NO: 14 and SEQ ID NO: 15, respectively, and CDR L1, CDR L2 and CDR L3 comprising SEQ ID NO: 16, SEQ ID NO: 17 and SEQ ID NO: 18, respectively;
c) CDR HI, CDR H2 and CDR H3 comprising SEQ ID NO: 19, SEQ ID NO: 20 and SEQ ID NO: 21, respectively, and CDR L1, CDR L2 and CDR L3 comprising SEQ ID NO: 22, SEQ ID NO: 23 and SEQ ID NO: 24, respectively;
d) CDR HI, CDR H2 and CDR H3 comprising SEQ ID NO: 64, SEQ ID NO: 65 and SEQ ID NO: 66, respectively, and CDR L1, CDR L2 and CDR L3 comprising SEQ ID NO: 67, SEQ ID NO: 68 and SEQ ID NO: 69, respectively;
e) CDR H1,CDR H2 and CDR H3 comprising SEQ ID NO: 70, SEQ ID NO: 71 and SEQ ID NO: 72, respectively, and CDR L1, CDR L2 and CDR L3 comprising SEQ ID NO: 73, SEQ ID NO: 74 and SEQ ID NO: 75, respectively;
f) CDR HI, CDR H2 and CDR H3 comprising SEQ ID NO: 76, SEQ ID NO: 77 and SEQ ID NO: 78, respectively, and CDR L1, CDR L2 and CDR L3 comprising SEQ ID NO: 79, SEQ ID NO: 80 and SEQ ID NO: 81, respectively; or
g) CDR HI, CDR H2 and CDR H3 comprising SEQ ID NO: 82, SEQ ID NO: 83 and SEQ ID NO: 84, respectively, and CDR L1, CDR L2 and CDR L3 comprising SEQ ID NO: 85, SEQ ID NO: 86 and SEQ ID NO: 87, respectively.

In another aspect, the present invention relates to an anti-PD-L1 antibody or antigen binding fragment thereof, wherein the anti-PD-L1 antibody comprises a heavy chain variable region (VH) comprising:
a) an amino acid sequence selected from SEQ ID NO: 1, SEQ ID NO: 3, SEQ ID NO: 5, SEQ ID NO: 56, SEQ ID NO: 58, SEQ ID NO: 60 or SEQ ID NO: 62;
b) an amino acid sequence that is at least 60%, such as at least 65%, at least 70%, at least 75%, at least 80%, at least 85%, at least 90%, at least 95%, at least 98%, or at least 99% identical to the amino acid sequence of a); or
c) an amino acid sequence with one or more amino acid modifications in the amino acid sequence of a), such as 1-5, 5-10, 10-20, 20-30 or 30-40 amino acid modifications, such as 1-40, 1-30, 1-20, 1-10 or 1-5 amino acid modifications.

As used herein, "amino acid modification" refers to the addition, substitution, insertion and/or deletion of one or more amino acids in the polypeptide chain.

In some embodiments, the anti-PD-L1 antibody further comprises a light chain variable region (VL) comprising:
a) an amino acid sequence selected from SEQ ID NO: 2, SEQ ID NO: 4, SEQ ID NO: 6, SEQ ID NO: 57, SEQ ID NO: 59, SEQ ID NO: 61 or SEQ ID NO: 63;
b) an amino acid sequence that is at least 60%, such as at least 65%, at least 70%, at least 75%, at least 80%, at least 85%, at least 90%, at least 95%, at least 98%, or at least 99% identical to the amino acid sequence of a); or
c) an amino acid sequence with one or more amino acid modifications in the amino acid sequence of a), such as 1-5, 5-10 or 10-20 amino acid modifications, such as 1-20, 1-10 or 1-5 amino acid modifications.

In some embodiments, the anti-PD-L1 antibody comprises the following VH and VL:
a) VH and VL comprising the amino acid sequences of SEQ ID NO: 1 and SEQ ID NO: 2, respectively;
b) VH and VL comprising an amino acid sequence that is at least 60%, such as at least 65%, at least 70%, at least 75%, at least 80%, at least 85%, at least 90%, at least 95%, at least 98%, or at least 99% identical to SEQ ID NO: 1 and SEQ ID NO: 2, respectively; or
c) VH and VL comprising the amino acid sequence with one or more amino acid modifications in SEQ ID NO: 1 and SEQ ID NO: 2, respectively.

In other embodiments, the anti-PD-L1 antibody comprises the following VH and VL:
a) VH and VL comprising the amino acid sequences of SEQ ID NO: 3 and SEQ ID NO: 4, respectively;
b) VH and VL comprising an amino acid sequence that is at least 60%, such as at least 65%, at least 70%, at least 75%, at least 80%, at least 85%, at least 90%, at least 95%, at least 98%, or at least 99% identical to SEQ ID NO: 3 and SEQ ID NO: 4, respectively; or
c) VH and VL comprising the amino acid sequence with one or more amino acid modifications in SEQ ID NO: 3 and SEQ ID NO: 4, respectively.

In some embodiments, the anti-PD-L1 antibody comprises the following VH and VL:
a) VH and VL comprising the amino acid sequences of SEQ ID NO: 5 and SEQ ID NO: 6, respectively;
b) VH and VL comprising an amino acid sequence that is at least 60%, such as at least 65%, at least 70%, at least 75%, at least 80%, at least 85%, at least 90%, at least 95%, at least 98%, or at least 99% identical to SEQ ID NO: 5 and SEQ ID NO: 6, respectively; or
c) VH and VL comprising the amino acid sequence with one or more amino acid modifications in SEQ ID NO: 5 and SEQ ID NO: 6, respectively.

In some embodiments, the anti-PD-L1 antibody comprises the following VH and VL:
a) VH and VL comprising the amino acid sequences of SEQ ID NO: 56 and SEQ ID NO: 57, respectively;
b) VH and VL comprising an amino acid sequence that is at least 60%, such as at least 65%, at least 70%, at least 75%, at least 80%, at least 85%, at least 90%, at least 95%, at least 98%, or at least 99% identical to SEQ ID NO: 56 and SEQ ID NO: 57, respectively; or
c) VH and VL comprising the amino acid sequence with one or more amino acid modifications in SEQ ID NO: 56 and SEQ ID NO: 57, respectively.

In other embodiments, the anti-PD-L1 antibody comprises the following VH and VL:
a) VH and VL comprising the amino acid sequences of SEQ ID NO: 58 and SEQ ID NO: 59, respectively;
b) VH and VL comprising an amino acid sequence that is at least 60%, such as at least 65%, at least 70%, at least 75%, at least 80%, at least 85%, at least 90%, at least 95%, at least 98%, or at least 99% identical to SEQ ID NO: 58 and SEQ ID NO: 59, respectively; or
c) VH and VL comprising the amino acid sequence with one or more amino acid modifications in SEQ ID NO: 58 and SEQ ID NO: 59, respectively.

In some embodiments, the anti-PD-L1 antibody comprises the following VH and VL:
a) VH and VL comprising the amino acid sequences of SEQ ID NO: 60 and SEQ ID NO: 61, respectively;
b) VH and VL comprising an amino acid sequence that is at least 60%, such as at least 65%, at least 70%, at least 75%, at least 80%, at least 85%, at least 90%, at least 95%, at least 98%, or at least 99% identical to SEQ ID NO: 60 and SEQ ID NO: 61, respectively; or
c) VH and VL comprising the amino acid sequence with one or more amino acid modifications in SEQ ID NO: 60 and SEQ ID NO: 61, respectively.

In some embodiments, the anti-PD-L1 antibody comprises the following VH and VL:
a) VH and VL comprising the amino acid sequences of SEQ ID NO: 62 and SEQ ID NO: 63, respectively;
b) VH and VL comprising an amino acid sequence that is at least 60%, such as at least 65%, at least 70%, at least 75%, at least 80%, at least 85%, at least 90%, at least 95%, at least 98%, or at least 99% identical to SEQ ID NO: 62 and SEQ ID NO: 63, respectively; or
c) VH and VL comprising the amino acid sequence with one or more amino acid modifications in SEQ ID NO: 62 and SEQ ID NO: 63, respectively.

In any embodiment of the aforementioned anti-PD-L1 antibody or antigen binding fragment thereof, wherein the anti-PD-L1 antibody can be a murine antibody, a chimeric antibody, a humanized antibody or a fully human antibody.

In any embodiment of the aforementioned anti-PD-L1 antibody or antigen binding fragment thereof, when an amino acid modification is involved, the amino acid modification can be located in a framework region of the variable region. In some embodiments, the amino acid modification is humanization.

In one aspect, the present invention relates to humanized anti-PD-L1 antibodies of the present invention.

In some embodiments, the humanized anti-PD-L1 antibody comprises VH comprising the amino acid sequence selected from SEQ ID NO: 39, SEQ ID NO: 40, SEQ ID NO: 41 or SEQ ID NO: 42, and VL comprising an amino acid sequence selected from SEQ ID NO: 43, SEQ ID NO: 44 or SEQ ID NO: 45.

In other embodiments, the humanized anti-PD-L1 antibody comprises VH comprising the amino acid sequence selected from SEQ ID NO: 31, SEQ ID NO: 32, SEQ ID NO: 33 or SEQ ID NO: 34, and VL comprising an amino acid sequence selected from SEQ ID NO: 35, SEQ ID NO: 36, SEQ ID NO: 37 or SEQ ID NO: 38.

In some embodiments, the antibody comprises VH comprising the amino acid sequence selected from SEQ ID NO: 46, SEQ ID NO: 47 or SEQ ID NO: 48, and VL comprising an amino acid sequence selected from SEQ ID NO: 49, SEQ ID NO: 50 or SEQ ID NO: 51.

In some embodiments of the aforementioned anti-PD-L1 antibody or antigen binding fragment thereof, the antibody may further comprise an Fc region amino acid modification which reduces the antibody-dependent cell-mediated cytotoxicity (ADCC) and/or complement-dependent cytotoxicity (CDC). In some embodiments, the modification comprises an N297A mutation.

In any embodiment of the aforementioned anti-PD-L1 antibody or antigen binding fragment thereof, the antibody may be selected from an IgG, an IgA, an IgM, an IgE or an IgD isotype. In some embodiments, the antibody is selected from IgG1, IgG2, IgG3, or IgG4 subtypes.

In any embodiment of the aforementioned anti-PD-L1 antibody or antigen binding fragment thereof, the antigen binding fragment may be selected from a Fab fragment, a Fab' fragment, a Fd fragment, a Fd' fragment, a Fv fragment, a dAb Fragment, a F(ab')₂ fragment, a single chain fragment, a diabody or a linear antibody.

In another aspect, the present invention relates to a bispecific antibody. As used herein, "bispecific antibody" refers to an artificially designed antibody, which is composed of two components with different antigen binding sites, and can bind to two different antigen binding sites at the same time.

In some embodiments, the bispecific antibody comprises a first antigen binding region binding to PD-L1, and a second antigen binding region binding to a second antigen, and the first antigen binding region comprises the CDR HI, CDR H2 and CDR H3 and/or CDR L1, CDR L2 and CDR L3 of the anti-PD-L1 antibody of the present invention, or the VH and/or VL of the anti-PD-L1 antibody of the present invention. In some embodiments, the second antigen may be selected from a tumor-associated antigen or an immune checkpoint molecule.

Many tumor-associated antigens associated with specific cancer have been identified in the art. As used herein, the term "tumor-associated antigen" refers to an antigen that is differentially expressed by cancer cells and therefore can be used to target cancer cells. Tumor-associated antigens are antigens that can potentially stimulate an obvious tumor-specific immune response. Some of these antigens are encoded by normal cells, but are not necessarily expressed by normal cells. These antigens can be characterized as those that are usually silent (i.e., not expressed) in normal cells, those that are expressed only during certain stages of differentiation, and those that are expressed over time, such as embryonic and fetal antigens. Other cancer antigens are encoded by mutant genes such as oncogenes (e.g. activated ras oncogene), suppressor genes (e.g. mutant p53), and fusion proteins produced by internal deletions or chromosomal translocations. Other cancer antigens can be encoded by viral genes, such as those carried on RNA and DNA tumor viruses. Many other tumor-associated antigens and antibodies against them are known and/or commercially available, and can also be produced by those skilled in the art.

Immune checkpoint protein receptors and ligands thereof (herein collectively referred to as immune checkpoint molecules) mediate the suppression of T cell-mediated cytotoxicity, and are usually expressed by tumors or expressed on nonreactive T cells in the tumor microenvironment, and allow tumors to evade immune attack. Inhibitors of the activity of immunosuppressive checkpoint protein receptors and ligands thereof can overcome the immunosuppressive tumor environment to allow the tumor's cytotoxic T cell attack. Examples of immune checkpoint proteins include, but are not limited to, PD-1, PD-L1, PD-L2, CTLA4, OX40, LAG3, TIM3, TIGIT and CD103. The regulation (including inhibition) of the activity of such proteins can be accomplished by immune checkpoint modulators, which can include, for example, antibodies, aptamers, small molecules, and soluble forms of checkpoint receptor proteins that target checkpoint proteins. Antibodies specific for PD-1, PD-L2, CTLA4, OX40, LAG3, TIM3, TIGIT and CD103 are known and/or commercially available, and can also be produced by those skilled in the art.

In one aspect, the present invention relates to a nucleotide sequence. In some embodiments, the nucleotide sequence encodes the polypeptide chain of the anti-PD-L1 antibody of the present invention. In other embodiments, the nucleotide sequence encodes the VH or VL of the anti-PD-L1 antibody of the present invention.

In another aspect, the present invention relates to a vector comprising the nucleotide sequence of the present invention.

As used herein, "vector" refers to a nucleic acid delivery vehicle into which polynucleotides can be inserted. When the vector allows for the expression of the protein encoded by the inserted polynucleotide, the vector is called an expression vector. A vector can be introduced into a host cell by transformation, transduction, or transfection, and then the genetic substance elements carried by the vector can be expressed in the host cell. Vectors are well known to those skilled in the art, including, but not limited to: (1) a plasmid; (2) a phagemid; (3) a cosmid; (4) an artificial chromosome, such as yeast artificial chromosome, bacterial artificial chromosome or artificial chromosome derived from PI; (5) a bacteriophage such as lambda bacteriophage or M13 bacteriophage and (6) an animal virus, such as retrovirus, adenovirus, adeno-associated virus, herpesvirus, poxvirus, and baculovirus. A vector can contain a variety of elements that control expression, including, but not limited to: a promoter sequence, a transcription initiation sequence, an enhancer sequence, a selection element, and reporter gene; in addition, the vector may further contain a replication initiation site.

In one aspect, the present invention relates to a recombinant host cell comprising the nucleotide sequence or vector of the present invention.

In another aspect, the present invention relates to a hybridoma cell producing the anti-PD-L1 antibody or antigen binding fragment thereof of the present invention.

In one aspect, the present invention relates to a conjugate comprising the anti-PD-L1 antibody or antigen binding fragment thereof, or the bispecific antibody of the present invention, and a moiety conjugated thereto. In some embodiments, the moiety may be selected from cytotoxins, radioisotopes, fluorescent labels, luminescent substances, chromogenic substances or enzymes.

In another aspect, the present invention relates to a composition comprising the anti-PD-L1 antibody or antigen binding fragment thereof, the bispecific antibody, or the conjugate of the present invention, and optionally one or more pharmaceutically acceptable carriers, excipients and/or diluents.

The phrase "pharmaceutically acceptable" refers to those compounds, materials, compositions and/or dosage forms that are suitable for use in contact with human and animal tissues within the scope of reasonable medical judgment without excessive toxicity, irritation, allergic response, or other problems or complications, and are commensurate with a reasonable benefit/risk ratio. As used herein, the phrase "pharmaceutically acceptable carriers, excipients and/or diluents" refers to pharmaceutically acceptable materials, compositions or vehicles, such as liquid or solid fillers, diluents, excipients, solvents, media, encapsulating materials, manufacturing aids or solvent encapsulating materials, which are related to maintaining the stability, solubility or activity of the anti-PD-L1 antibody or antigen binding fragment thereof of the present invention.

In one aspect, the present invention relates to a method for treating a disease in a subject comprising administering to the subject the anti-PD-L1 antibody or antigen binding fragment thereof, bispecific antibody, conjugate or composition of the present invention.

In another one aspect, the present invention relates to use of the anti-PD-L1 antibody or antigen binding fragment thereof, bispecific antibody, conjugate or composition of the present invention in the treatment of a disease in a subject.

In yet another aspect, the present invention relates to use of the anti-PD-L1 antibody or antigen binding fragment thereof, bispecific antibody, conjugate or composition of the present invention in the preparation of a medicament for treating a disease in a subject.

In some embodiments of the above methods and use, the disease may be cancer. Specific examples of cancer include, but are not limited to: basal cell carcinoma, cholangiocarcinoma; bladder cancer; bone cancer; breast cancer; peritoneal cancer; cervical cancer; cholangiocarcinoma; choriocarcinoma; colon and rectal cancer; connective tissue cancer; digestive system cancer; endometrial cancer; esophageal cancer; eye cancer; head and neck cancer; gastric cancer; glioblastoma; liver cancer; kidney cancer; laryngeal cancer; leukemia; liver cancer; lung cancer (e.g., small cell lung cancer, non-small cell lung cancer, lung adenocarcinoma, and lung squamous cell carcinoma); lymphoma, including Hodgkin's lymphoma and non-Hodgkin's lymphoma; melanoma; myeloma; neuroblastoma; oral cancer; ovarian cancer; pancreatic cancer; prostate cancer; retinoblastoma; rhabdomyosarcoma; rectal cancer; respiratory cancer; salivary gland cancer; sarcoma; skin cancer; squamous cell carcinoma; testicular cancer; thyroid cancer; uterine or endometrial cancer; urinary system cancer; B-cell lymphoma; chronic lymphocytic leukemia (CLL); acute lymphoblastic leukemia (ALL); hairy cell leukemia; chronic myeloblastic leukemia, and the like.

In some embodiments, the cancer is selected from melanoma, lymphoma, bladder cancer, non-small cell lung cancer, head and neck cancer, colon cancer or skin cancer.

In some embodiments of the above methods and use, further comprising administering one or more additional therapeutic agents to the subject. In some embodiments, the therapeutic agent is selected from an antibody, a chemotherapeutic drug, or a small molecule compound. In some embodiments, the therapeutic agent targets a tumor-associated antigen. In other embodiments, the therapeutic agent targets an immune checkpoint molecule which may be selected from PD-1, PD-L2, CTLA4, OX40, LAG3, TIM3, TIGIT, or CD103.

As used herein, the term "chemotherapeutic agent" refers to any chemical agent that has therapeutic usefulness in the treatment of a disease characterized by abnormal cell growth. Chemotherapeutic agents as used herein include chemical agents and biological agents. These agents function to inhibit the cell activity that cancer cells depend on for continued survival. The categories of chemotherapeutic agents include alkylating/alkaloid agents, antimetabolites, hormones or hormone analogs, as well as various antibiotic drugs.

### Brief Description of the Drawings

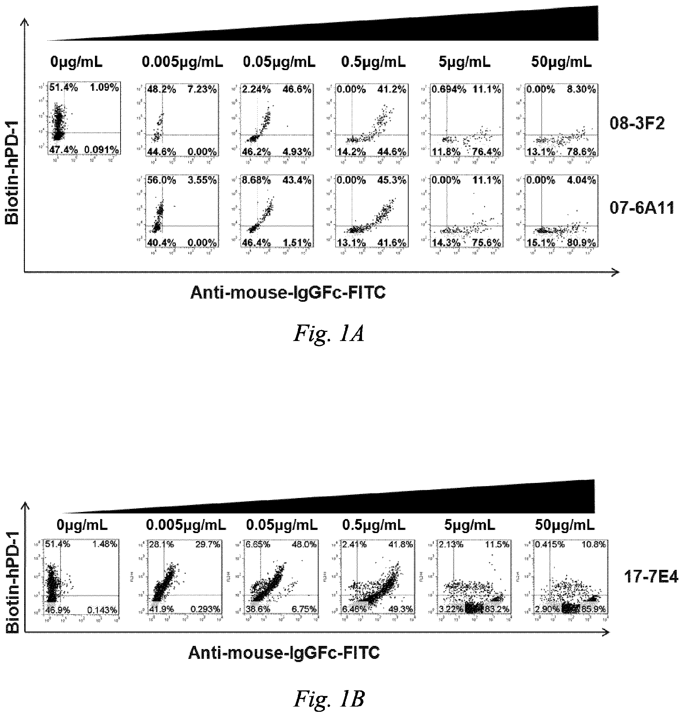

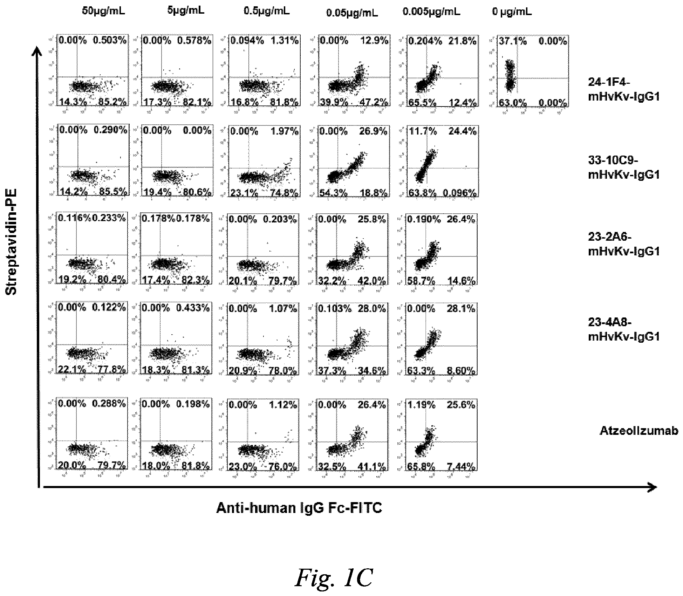

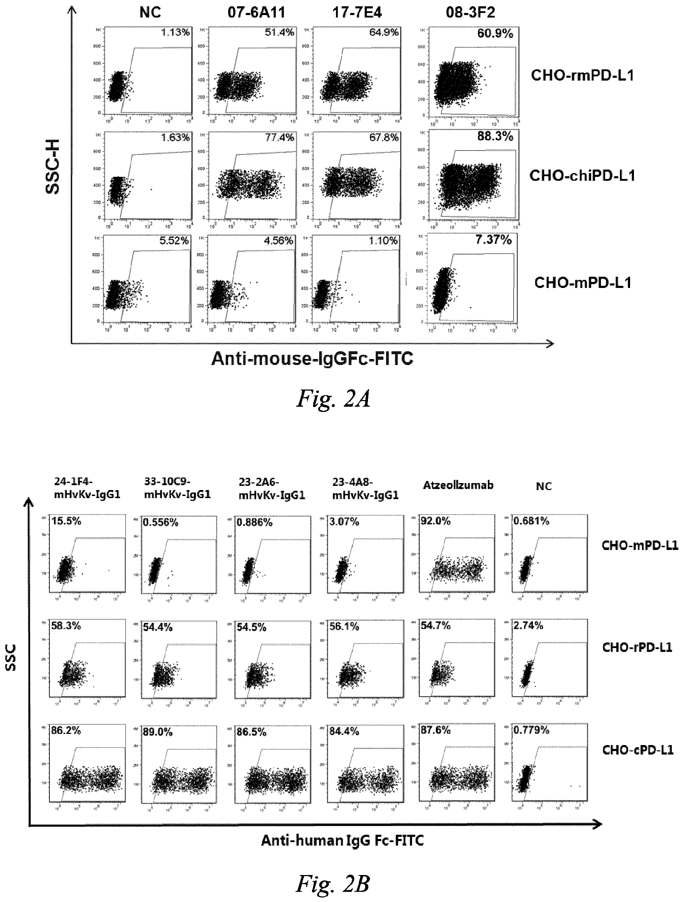

The flow cytometry results in Figure 1 show the effect of the anti-PD-L1 antibody of the present invention in blocking the binding between biotin-hPD1 ligand and PD-L1. Figure 1A shows the results of antibodies 08-3F2 and 07-6A11; Figure 1B shows the results of antibody 17-7E4; Figure 1C shows the results of chimeric antibodies 24-1F4-mHvKv-IgG1, 33-10C9-mHvKv-IgG1, 23-2A6-mHvKv-IgG1 and 23-4A8-mHvKv-IgG1. Atzeolizumab is used as a positive control.
The flow cytometry of Figure 2 shows the cross-reactions between the anti-PD-L1 antibody of the present invention and PD-L1 proteins and chimeric PD-L1 proteins derived from different species. Figure 2A shows the results of antibodies 07-6A11, 17-7E4 and 08-3F2; Figure 2B shows the results of chimeric antibodies 24-1F4-mHvKv-IgG1, 33-10C9-mHvKv-IgG1, 23-2A6-mHvKv-IgG1 and 23-4A8-mHvKv-IgG1. NC: negative control. Atzeolizumab is used as a positive control.

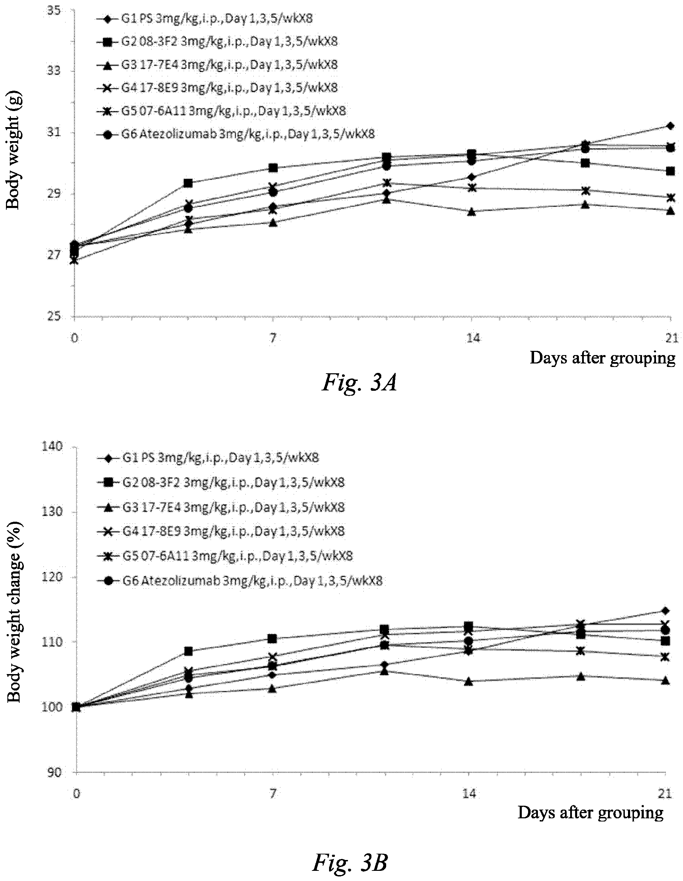

Figure 3 shows a line graph showing the change in body weight of experimental animals over time after treatment with the indicated anti-PD-L1 antibody. Figure 3A shows the change of the absolute value of experimental animals' body weight over time. Figure 3B shows the percentage change in the body weight of experimental animals over time. Physiological saline (PS) is used as a negative control. Atzeolizumab is used as a positive control.

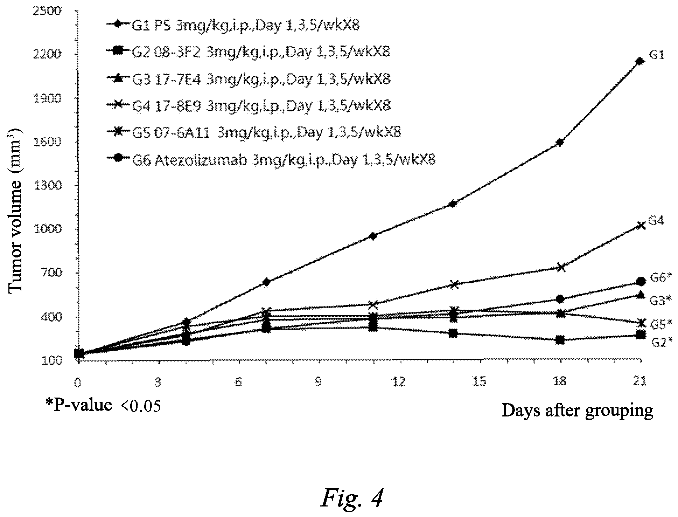

Figure 4 shows a line graph showing the change in tumor volume in experimental animals over time after treatment with the indicated anti-PD-L1 antibody. Physiological saline (PS) is used as a negative control. Atzeolizumab is used as a positive control.

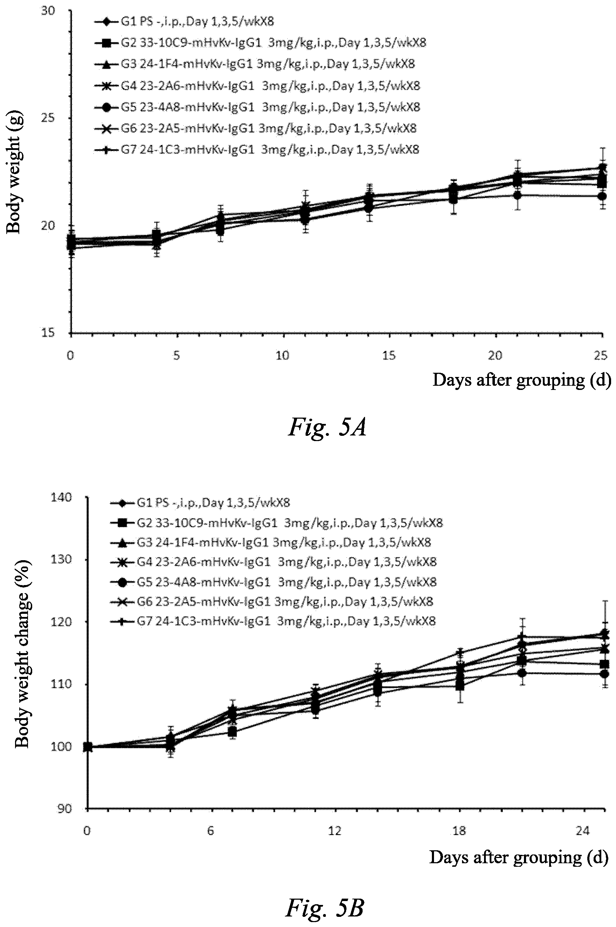

Figure 5 shows a line graph showing the change in body weight of experimental animals over time after treatment with the indicated anti-PD-L1 antibody. Figure 5A shows the change of the absolute value of experimental animals' body weight over time. Figure 5B shows the percentage change in the body weight of experimental animals over time. Physiological saline (PS) is used as a negative control.

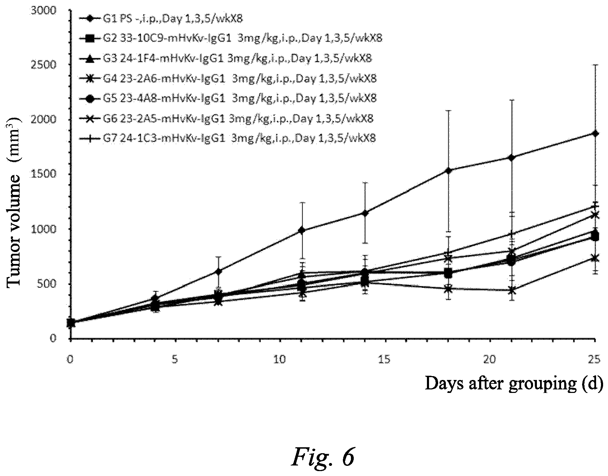

Figure 6 shows a line graph showing the change in tumor volume in experimental animals over time after treatment with the indicated anti-PD-L1 antibody. Physiological saline (PS) is used as a negative control.

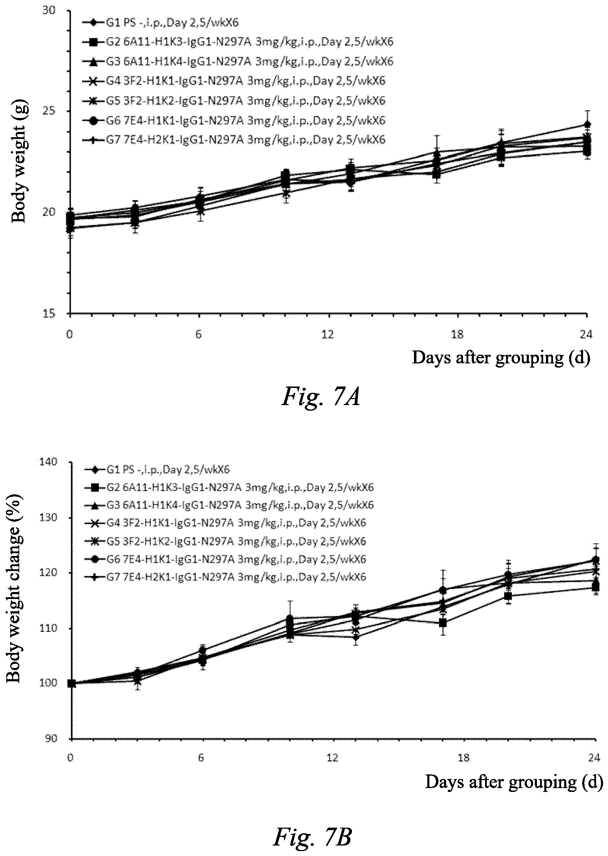

Figure 7 shows a line graph showing the change in body weight of experimental animals over time after treatment with the indicated anti-PD-L1 antibody. Figure 7A shows the change of the absolute value of experimental animals' body weight over time. Figure 7B shows the percentage change in the body weight of experimental animals over time. Physiological saline (PS) is used as a negative control.

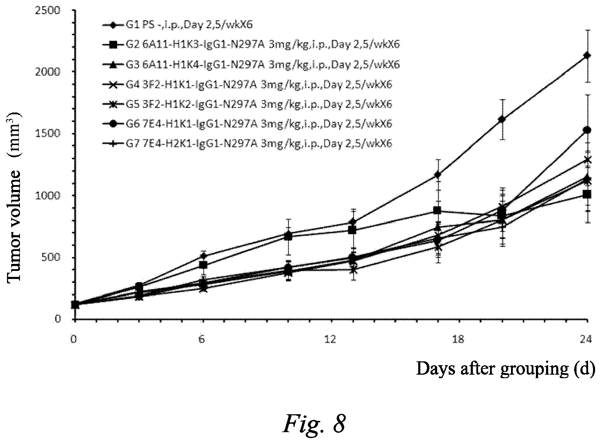

Figure 8 shows a line graph showing the change in tumor volume in experimental animals over time after treatment with the indicated anti-PD-L1 antibody. Physiological saline (PS) is used as a negative control.

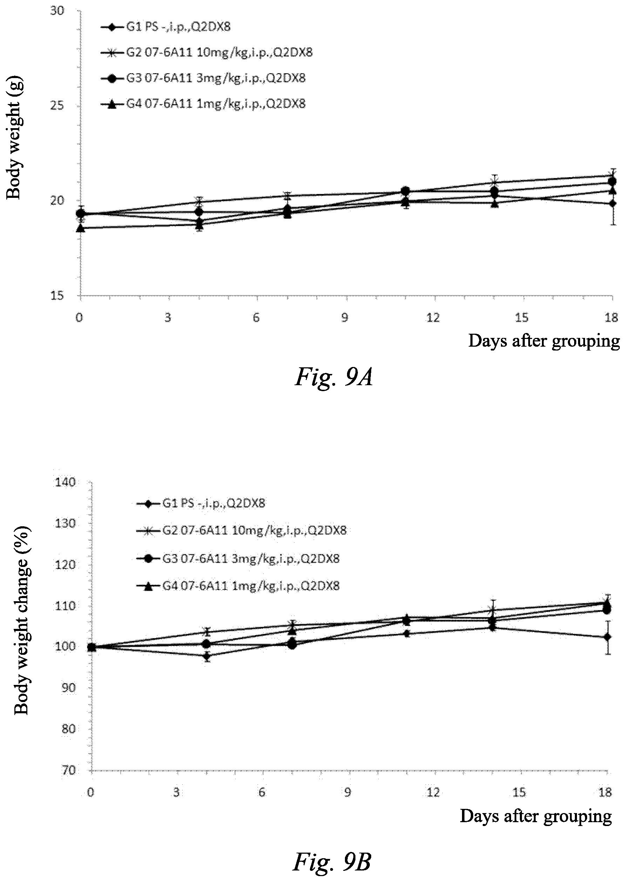

Figure 9 shows a line graph showing the change in body weight of experimental animals over time after treatment with different doses of the anti-PD-L1 antibody. Figure 9A shows the change of the absolute value of experimental animals' body weight over time. Figure 9B shows the percentage change in the body weight of experimental animals over time. Physiological saline (PS) is used as a negative control.

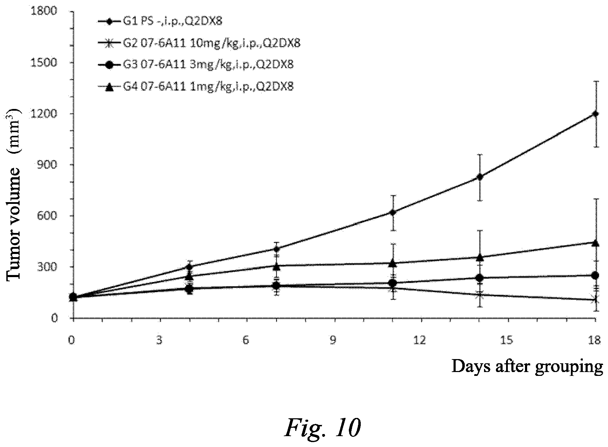

Figure 10 shows a line graph showing the change in tumor volume in experimental animals over time after treatment with different doses of the anti-PD-L1 antibody. Physiological saline (PS) is used as a negative control.

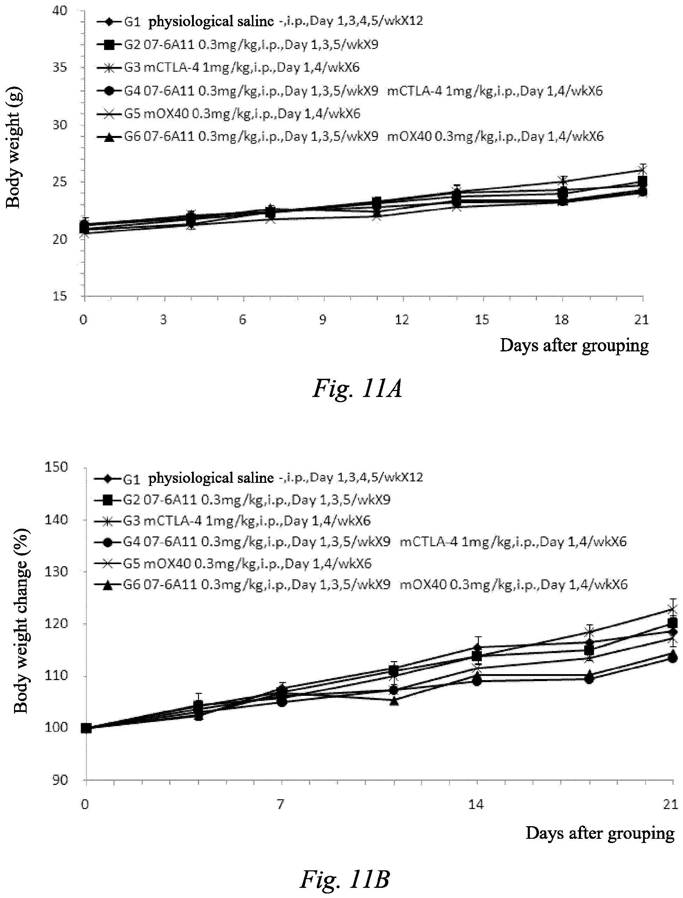

Figure 11 shows a line graph showing the change in body weight of experimental animals over time after treatment with the indicated anti-PD-L1 antibody, anti-CTLA-4 antibody, anti-OX40 antibody or a combination thereof. Figure 11A shows the change of the absolute value of experimental animals' body weight over time. Figure 11B shows the percentage change in the body weight of experimental animals over time. Physiological saline is used as a negative control.

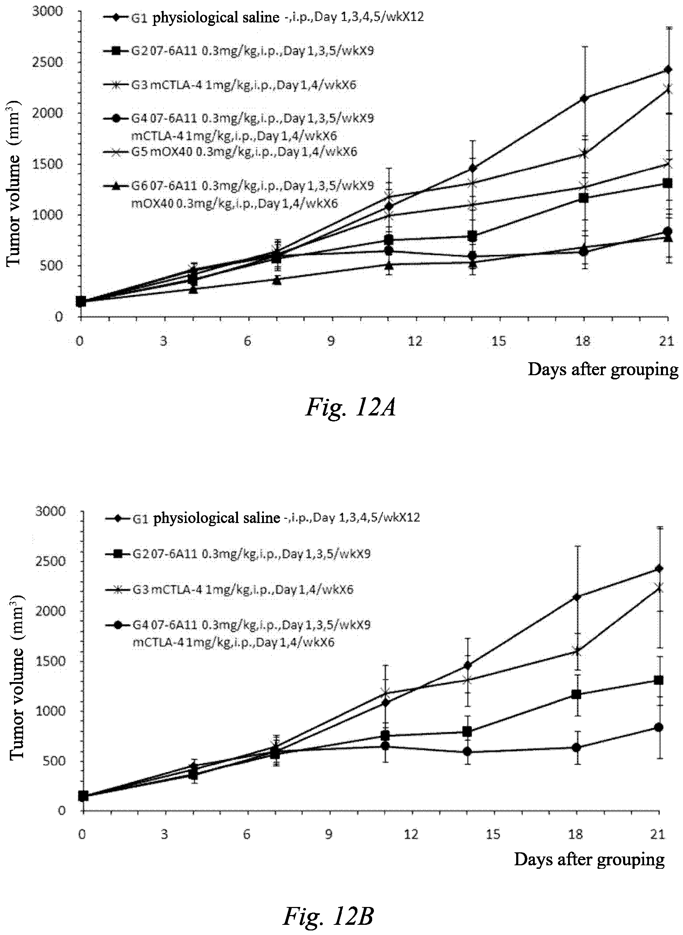

Figures 12A to 12C show line graphs showing the change in tumor volume in experimental animals over time after treatment with the indicated anti-PD-L1 antibody, anti-CTLA-4 antibody, anti-OX40 antibody or a combination thereof. Physiological saline is used as a negative control.

### Detailed Description of Embodiments

Hereinafter, the content of the present invention will be further described in conjunction with examples. It should be understood that the following examples are only illustrative and should not be considered as limiting the scope of the present invention.

The name of the antibody used herein includes information of the antibody source, variable region and constant region. For example, the chimeric antibody 08-3F2-mHvKv-IgG1 is an antibody composed of the VH and VL regions of the murine antibody 08-3F2 ("3F2") and the constant region of human IgG1; similarly, the humanized antibody 3F2-H1K2-IgG1 is an antibody composed of the humanized heavy chain variable region H1 and light chain variable region K2 of the murine antibody 08-3F2 ("3F2"), and the constant region of human IgG1 . Some antibodies further have an N297A mutation at the Fc terminal (e.g., 23-2A6-mHvKv-IgG1-N297A) to eliminate the Fc functional effect by mutating glycosylation sites.

### Example 1. Generating of the antibody

The anti-PD-L1 monoclonal antibodies 08-3F2 ("3F2"), 07-6A11("6A11"), 17-7E4 ("7E4"), 23-2A6 ("2A6"), 23-4A8 ("4A8"), 33-10C9 ("10C9") and 24-1F4 ("1F4") of the present invention were obtained by immunizing mice with a recombinant human PD-L1 protein or an expression plasmid encoding the recombinant human PD-L1 protein, and then constructing hybridoma cells. GE AKTA protein chromatography automatic purifier (GE Healthcare) was used for antibody purification according to the manufacturer's instructions. The amino acid sequences of the heavy chain variable region (VH), light chain variable region (VL) and CDR of the above antibodies were shown in Tables 1 to 3 below.

**Table 1. VH and VL sequences of the antibodies**

| | | |
|---|---|---|
| 08-3F2 ("3F2") Heavy chain variable region | DVQLQESGPDLVKPSQTLSLTCTVTGYSITSG YNWHWIRQFPGNKLEWMGYIHHSSITNYNP SLKSRISITRDTSKNQFFLQLSSVTTEDTATFY CAREGYDYDWFAYWGQGTLVTVSA | SEQ ID NO: 1 |
| 08-3F2 ("3F2") Light chain variable region | DIVLAQSPATLSVTPGDSVSLSCRASQSISNN LHWYQQKSHESPRLLIKYASQSISGIPSRFSG SGSGTDFTLSINSVETEDFGMYFCQQSKSWP FTFGSGTRLEIK | SEQ ID NO: 2 |
| 07-6A11 ("6A11") Heavy chain variable region | DVQLVESGGGLVQPGGSRKLSCAASGFTFSS FGMHWVRQAPEKGLEWVAYISSGSNTIYYA DTVKGRFTISRDNPKNTLFLQMTSLRSEDTAI YYCTRNGYDGWYAMDYWGQGTSVTVSS | SEQ ID NO: 3 |
| 07-6A11 ("6A11") Light chain variable region | QIVLTQSPAIMSASPGEKVTMTCSASSSVSYI HWFQQKSGTSPKRWIYDTSKLASGVPARFS GRGSGTSYSLTISSMEAEDAATYYCQQWST NPFTFGSGTKLEIK | SEQ ID NO: 4 |
| 17-7E4 ("7E4") Heavy chain variable region | QVQLQQSGAELVRPGVSVKISCKGSGFKFTD YAIHWVKQSHAKSLEWIGVISIYYGEASYNQ KFKDKATLTVDTSSSTAYMELARLTSEDSAIY YCAREDYYGSSSYFDYWGQGTALTVSS | SEQ ID NO: 5 |
| 17-7E4 ("7E4") Light chain variable region | ENVLTQSPAIMAASLGQKVTMTCSASSSVSS SYLHWYQQKSGASPKPLIHRTSNLASGVPAR FSGSGSGTSYSLTISSVEAEDDATYYCQQWS GYPYTFGGGTKLEIK | SEQ ID NO: 6 |
| 23-2A6 ("2A6") Heavy chain variable region | QVQLKESGPGLVAPSQSLSITCTVSGFSLTSY DISWIRQPPGKGLEWLGAIWTGGGTNYNSAF MSRLSISKDNSKSQVFLKMNSLQTDDTAIYY CVRDPYYYAMDYWGQGTSVTVSS | SEQ ID NO: 56 |
| 23-2A6 ("2A6") Light chain variable region | DIQMNQSPSSLSASLGDTITITCHASQNLNVW LSWYQQKPGNIPKLLIYKASNLHTGVPSRFS GSGSGTGFTLTISSLQPEDIATYYCQQGQSYP RTFGGGTKLEIK | SEQ ID NO: 57 |
| 23-4A8 ("4A8") Heavy chain variable region | EVQLQESGPSLVKPSQTLSLTCSVTGDSITSG YWNWIRKFPGNKLEYMGYISYTGSTYYNPS LKSRFSITRDTSKNQYFLQLNSVTTEDTATY YCASYEGWLLPFAYWGQGTLVTVSA | SEQ ID NO: 58 |
| 23-4A8 ("4A8") Light chain variable region | DIVMSQSPSSLAVSVGEKVPLSCKSSQSLLYS SNQKSSLAWYQQKPGQSPKLLIYWASTRES GVPDRFTGSGSGTDFTLTISSVKAEDLAVYY CQQYYGYPFTFGSGTKLEIK | SEQ ID NO: 59 |
| 33-10C9 ("10C9") Heavy chain variable region | QVQLQQPGAELVKPGSSVKLSCKASGYTFTT YYIYWVKQRPGQGLEWIGGINPYNGGTSFN EKFESKATLTVDISSSTAFMQLSSLTSEDSAV YYCTRDGNYVDYWGQGTTLTVSS | SEQ ID NO: 60 |
| 33-10C9 ("10C9") Light chain variable region | QIVLTQSPAIMSASPGEKVTITCSASSTVSYV HWLQQKPDTSPKLWIYSTSNLASGVPARFSG SGSGTSYSLTISRMEAEDAATYYCQQRSSSPP TFGSGTKLEIK | SEQ ID NO: 61 |
| 24-1F4 ("1F4") Heavy chain variable region | EVQLRESGPSLVRPSQTLSLTCSVTGDSFTSG YWNWIRKFPGNELESMGYISYSGSTYYNPSL KSRISITRDTSKSQFYLQLSSVTAEDTATYYC ARSEGWLLPFAYWGQGTLVTVSA | SEQ ID NO: 62 |
| 24-1F4 ("1F4") Light chain variable region | DIVMSQSPSSLPVSVGEKVTMSCKSSQSLLYS SNQKNSLAWYQQKPGQSPKLLIYWASTRES GVPDRFTGSGSGTDFTLTISSVQAEDLSVYY CQQYYGYPFTFGSGTKLEIK | SEQ ID NO: 63 |

**Table 2. Kabat CDR**

| **Anti body** | **VH CD R1** | **SEQ ID NO:** | **VH CD R2** | **SEQ ID NO:** | **VH CD R3** | **SEQ ID NO:** | **VL CD R1** | **SEQ ID NO:** | **VL CD R2** | **SEQ ID NO:** | **VL CDR 3** | **SEQ ID NO:** |
|---|---|---|---|---|---|---|---|---|---|---|---|---|
| 07-6A11 | SFG MH | 7 | YIS SGS NTI YY ADT VK G | 8 | NG YD GW YA MD Y | 9 | SAS SSV SYI H | 10 | DTS KLA S | 11 | QQ WST NPF T | 12 |
| 08-3F2 | SGY NW H | 13 | YIH HSS ITN YNP SLK S | 14 | EGY DY DWF AY | 15 | RAS QSI SNN LH | 16 | YAS QSI S | 17 | QQS KSW PFT | 18 |
| 17-7E4 | DY AIH | 19 | VISI YY GEA SYN QKF KD | 20 | EDY YGS SSY FDY | 21 | SAS SSV SSS YLH | 22 | RTS NLA S | 23 | QQ WSG Y PYT | 24 |
| 23-2A6 | SYD IS | 64 | AIW TGG GTN YNS AF MS | 65 | DPY YY AM DY | 66 | HAS QNL NV WL S | 67 | KAS NLH T | 68 | QQG QSY PRT | 69 |
| 23-4A8 | SGY WN | 70 | YIS YTG STY YNP SLK S | 71 | YEG WL LPF AY | 72 | KSS QSL LYS SNQ KSS LA | 73 | WAS TRE S | 74 | QQY YGY PFT | 75 |
| 33-10C9 | TYY IY | 76 | GIN PYN GGT SFN EKF ES | 77 | DG NY VD Y | 78 | SAS STV SYV H | 79 | STS NLA S | 80 | QQR SSSP PT | 81 |
| 24-1F4 | SGY WN | 82 | YIS YSG STY YNP SLK S | 83 | SEG WL LPF AY | 84 | KSS QSL LYS SNQ KNS LA | 85 | WAS TRE S | 86 | QQY YGY PFT | 87 |

**Table 3. Chothia CDR**

| **Anti body** | **VH CD R1** | **SEQ ID NO:** | **VH CD R2** | **SEQ ID NO:** | **VH CD R3** | **SEQ ID NO:** | **VL CD R1** | **SEQ ID NO:** | **VL CD R2** | **SEQ ID NO:** | **VL CDR 3** | **SEQ ID NO:** |
|---|---|---|---|---|---|---|---|---|---|---|---|---|
| 07-6A1 1 | GFT FSS FG MH | 25 | SSG SNT | 26 | NG YD GW YA MD Y | 9 | SAS SSV SYI H | 10 | DTS KLA S | 11 | QQ WST NPF T | 12 |
| 08-3F2 | GY SIT SGY NW H | 27 | HHS SI | 28 | EGY DY DWF AY | 15 | RAS QSI SNN LH | 16 | YAS QSI S | 17 | QQS KSW PFT | 18 |
| 17-7E4 | GF KFT DY AIH | 29 | SIY YGE | 30 | EDY YGS SSY FDY | 21 | SAS SSV SSS YLH | 22 | RTS NLA S | 23 | QQ WSG Y PYT | 24 |
| 23-2A6 | GFS LTS YDI S | 88 | WTG GG | 89 | DPY YY AM DY | 90 | HAS QNL NV WL S | 91 | KAS NLH T | 92 | QQG QSY PRT | 93 |
| 23-4A8 | GD SIT SG YW N | 94 | SYT GS | 95 | YEG WL LPF AY | 96 | KSS QSL LYS SNQ KSS LA | 97 | WAS TRE S | 98 | QQY YGY PFT | 99 |
| 33-10C9 | GY TFT TY YIY | 100 | NPY NG G | 101 | DG NY VD Y | 102 | SAS STV SYV H | 103 | STS NLA S | 104 | QQR SSSP PT | 105 |
| 24-1F4 | GD SFT SG YW N | 106 | SYS GS | 107 | SEG WL LPF AY | 108 | KSS QSL LYS SNQ KNS LA | 109 | WAS TRE S | 110 | QQY YGY PFT | 111 |

### Example 2. Humanization of antibodies

Then, the murine antibodies were humanized. Specifically, partial amino acid sequences of the heavy chain variable region and light chain variable region of the murine antibodies 6A11, 3F2 and 7E4 were replaced with humanized sequences to optimize the binding affinity with the antigen and improve the drug-like properties of these antibodies. For each antibody, more than one humanized heavy chain variable region and light chain variable region sequences were generated. The amino acid sequences of the humanized heavy chain variable region and light chain variable region were shown in Table 4 below.

**Table 4. Variable region sequences of the humanized antibodies**

| **Variable region of the humanized antibody** | **Amino acid sequence** | **SEQ ID NO:** |
|---|---|---|
| 6A11 Humanized heavy chain variable region (H1) | EVQLVESGGGLVQPGGSLRLSCAASGFTFSSFGMHWVR QAPGKGLEWVSYISSGSNTIYYADTVKGRFTISRDNAK NSLYLQMNSLRAEDTAVYYCARNGYDGWYAMDYWG QGTTVTVSS | 31 |
| 6A11 Humanized heavy chain variable region (H2) | EVQLVESGGGLVQPGGSLRLSCAASGFTFSSFGMHWVR QAPGKGLEWVSYISSGSNTIYYADTVKGRFTISRDNAK NSLYLQMNSLRAEDTAVYYCTRNGYDGWYAMDYWG QGTTVTVSS | 32 |
| 6A11 Humanized heavy chain variable region (H3) | EVQLVESGGGLVQPGGSRRLSCAASGFTFSSFGMHWVR QAPGKGLEWVAYISSGSNTIYYADTVKGRFTISRDNAK NSLYLQMNSLRAEDTAVYYCTRNGYDGWYAMDYWG QGTTVTVSS | 33 |
| 6A11 Humanized heavy chain variable region (H4) | EVQLVESGGGLVQPGGSRRLSCAASGFTFSSFGMHWVR QAPGKGLEWVAYISSGSNTIYYADTVKGRFTISRDNPK NSLYLQMNSLRAEDTAIYYCTRNGYDGWYAMDYWGQ GTTVTVSS | 34 |
| 6A11 Humanized light chain variable region (K1) | EIVLTQSPATLSLSPGERATLSCSASSSVSYIHWYQQKPG QAPRRLIYDTSKLASGIPARFSGSGSGTDFTLTISSLEPED FATYYCQQWSTNPFTFGQGTKLEIKR | 35 |
| 6A11 Humanized light chain variable region (K2) | EIVLTQSPATLSASPGERATLSCSASSSVSYIHWYQQKP GQAPRRLIYDTSKLASGIPARFSGSGSGTDYTLTISSLEP EDAATYYCQQWSTNPFTFGQGTKLEIKR | 36 |
| 6A11 Humanized light chain variable region (K3) | EIVLTQSPATLSASPGERATLSCSASSSVSYIHWFQQKPG QSPRRWIYDTSKLASGIPARFSGSGSGTDYTLTISSMEPE DFATYYCQQWSTNPFTFGQGTKLEIKR | 37 |
| 6A11 Humanized light chain variable region (K4) | EIVLTQSPATLSASPGERATLSCSASSSVSYIHWFQQKPG QSPRRWIYDTSKLASGIPARFSGSGSGTSYTLTISSMEPE DAATYYCQQWSTNPFTFGQGTKLEIKR | 38 |
| 3F2 Humanized heavy chain variable region (H1) | QVQLQESGPGLVKPSETLSLTCTVSGYSITSGYNWHWIR QFPGNGLEWMGYIHHSSITNYNPSLKSRITISRDTSKNQF SLKLSSVTAADTATYYCAREGYDYDWFAYWGQGTLV TVSS | 39 |
| 3F2 Humanized heavy chain variable region (H2) | DVQLQESGPGLVKPSETLSLTCTVSGYSITSGYNWHWIR QFPGNGLEWMGYIHHSSITNYNPSLKSRITISRDTSKNQF SLKLSSVTAADTATFYCAREGYDYDWFAYWGQGTLVT VSS | 40 |
| 3F2 Humanized heavy chain variable region (H3) | DVQLQESGPGLVKPSETLSLTCTVSGYSITSGYNWHWIR QFPGNGLEWMGYIHHSSITNYNPSLKSRITISRDTSKNQF SLKLSSVTAEDTATFYCAREGYDYDWFAYWGQGTLVT VSS | 41 |
| 3F2 Humanized heavy chain variable region (H4) | DVQLQESGPGLVKPSETLSLTCTVSGYSITSGYNWHWIR QFPGNKLEWMGYIHHSSITNYNPSLKSRITISRDTSKNQF FLQLSSVTAEDTATFYCAREGYDYDWFAYWGQGTLVT VSS | 42 |
| 3F2 Humanized light chain variable region (K1) | DIVLtQSPdfqSVTPkekVtitCRASQSISNNLHWYQQKpdES PkLLIKYASQSISGIPSRFSGSGSGTDFTLtINSVEaEDFaM YFCQQSKSWPFTFGqGTRLEIK | 43 |
| 3F2 Humanized light chain variable region (K2) | DIVLTQSPDFQSVTPKEKVTLSCRASQSISNNLHWYQQK PDESPKLLIKYASQSISGIPSRFSGSGSGTDFTLTINSVEA EDFAMYFCQQSKSWPFTFGQGTRLEIK | 44 |
| 3F2 Humanized light chain variable region (K3) | DIVLTQSPDTQSVTPKESVTLSCRASQSISNNLHWYQQK SHESPKLLIKYASQSISGIPSRFSGSGSGTDFTLTINSVEA EDFAMYFCQQSKSWPFTFGQGTRLEIK | 45 |
| 7E4 Humanized heavy chain variable region (H1) | QVQLVQSGAEVKKPGASVKVSCKASGFKFTDYAIHWV RQAPGQSLEWMGVISIYYGEASYNQKFKDRVTLTVDTS ASTAYMELSSLRSEDTAVYYCAREDYYGSSSYFDYWG QGTLVTVSS | 46 |
| 7E4 Humanized heavy chain variable region (H2) | QVQLVQSGAEVKKPGASVKVSCKASGFKFTDYAIHWV RQAPGKSLEWMGVISIYYGEASYNQKFKDRVTLTVDTS ASTAYMELSSLRSEDTAVYYCAREDYYGSSSYFDYWG QGTLVTVSS | 47 |
| 7E4 Humanized heavy chain variable region (H3) | QVQLVQSGAEVVKPGASVKISCKGSGFKFTDYAIHWVR QAPGKSLEWMGVISIYYGEASYNQKFKDKVTLTVDTSA STAYMELSSLRSEDTAIYYCAREDYYGSSSYFDYWGQG TLLTVSS | 48 |
| 7E4 Humanized light chain variable region (K1) | DNQLTQSPSFLSASVGDRVTITCSASSSVSSSYLHWYQQ KPGAAPKPLIHRTSNLASGVPSRFSGSGSGTEFTLTISSL QPEDFATYYCQQWSGYPYTFGGGTKLEIK | 49 |
| 7E4 Humanized light chain variable region (K2) | DNVLTQSPSFLSASVGDRVTMTCSASSSVSSSYLHWYQ QKPGASPKPLIHRTSNLASGVPSRFSGSGSGTEFTLTISSL QPEDFATYYCQQWSGYPYTFGGGTKLEIK | 50 |
| 7E4 Humanized light chain variable region (K3) | ENVLTQSPSFLSASVGDRVTMTCSASSSVSSSYLHWYQ QKSGASPKPLIHRTSNLASGVPSRFSGSGSGTEYTLTISS VQPEDFATYYCQQWSGYPYTFGGGTKLEIK | 51 |

### Example 3. Binding testing of antibodies

### Blocking the binding of PD-L1 and PD-1

This assay was used to determine whether the anti-PD-L1 antibodies can block the binding of PD-L1 and PD-1. The test was detailed as follows: a 96-well cell culture plate was used, and CHO-hPD-L1 cells expressing human PD-L1 protein (25 µ1, 2x10⁴ cells/well) were added to each well. The purified ascites were titrated to the concentration of 50 µg/ml, 5 µg/ml, 0.5 µg/ml, 0.05 µg/ml, and 0.005 µg/ml. 25 µ1 of serum diluted at 1 : 100 was added to each well at 4°C and incubated for 30 minutes. 50 µl of Biotin-hPD-1 protein diluted to 04µg/ml was added to each well at 4°C and incubated for 15 minutes. The 96-well cell culture plate was centrifuged at 1200 rpm for 5 minutes and washed twice with PBS. Subsequently, 50 µl of anti-mouse IgG Fc-FITC at 1 : 100 dilution and streptavidin-PE secondary antibody at 1 : 100 dilution were added to each well, and incubated at 4°C for 30 minutes. Subsequently, the 96-well cell culture plate was centrifuged at 1200 rpm for 5 minutes, and washed once with PBS. Then 200 µl of PBS was added to each well, and flow cytometry analysis was performed.

As shown in Figure 1A, Figure 1B and Figure 1C, when the concentration of the purified anti-PD-L1 antibodies (08-3F2, 07-6A11 and 17-7E4) and the chimeric anti-PD-L1 antibodies (24-1F4-mHvKv-IgG1, 33-10C9-mHvKv-IgG1, 23-2A6-mHvKv-IgG1, 23-4A8-mHvKv-IgG1) increased, the fluorescence intensity of the PE-labeled Biotin-hPD-1 decreased, suggesting that the binding between human PD-L1 and PD-1wasblocked by anti-PD-L1 antibodies of the present invention.

### Cross-reactivity testing of antibodies

The rhesus monkey PD-L1 protein coding sequence (rmPD-L1, SEQ ID NO: 54), the mouse PD-L1 protein coding sequence (mPD-L1, SEQ ID NO: 53), and the human-mouse chimeric PD-L1 (the extracellular region of the mouse PD-L1 protein was replaced with human protein fragments) protein coding sequence (chiPD-L1, SEQ ID NO: 55) were transferred into CHO cells for protein expression and used for the antibody cross-reactivity testing. The amino acid sequences of the above-mentioned PD-L1 proteins were shown in Table 5 below.

**Table 5. PD-L1 protein sequences of different species**

| **Protein** | **Amino acid sequence** | **SEQ ID NO:** |
|---|---|---|
| Human PD-L1 (hPD-L1) NP_054862 .1 | MRIFAVFIFM TYWHLLNAFT VTVPKDLYVV EYGSNMTIEC KFPVEKQLDL AALIVYWEME DKNIIQFVHG EEDLKVQHSS YRQRARLLKD QLSLGNAALQ ITDVKLQDAG VYRCMISYGG ADYKRITVKV NAPYNKINQR ILVVDPVTSE HELTCQAEGY PKAEVIWTSS DHQVLSGKTT TTNSKREEKL FNVTSTLRIN TTTNEIFYCT FRRLDPEENH TAELVIPELP LAHPPNERTH LVILGAILLC LGVALTFIFR LRKGRMMDVK KCGIQDTNSK KQSDTHLEET | 52 |
| Mouse PD-L1 (mPD-L1) NP_068693 .1 | MRIFAGIIFT ACCHLLRAFT ITAPKDLYVV EYGSNVTMEC RFPVERELDL LALVVYWEKE DEQVIQFVAG EEDLKPQHSN FRGRASLPKD QLLKGNAALQ ITDVKLQDAG VYCCIISYGG ADYKRITLKV NAPYRKINQR ISVDPATSEH ELICQAEGYP EAEVIWTNSD HQPVSGKRSV TTSRTEGMLL NVTSSLRVNA TANDVFYCTF WRSQPGQNHT AELIIPELPA THPPQNRTHW VLLGSILLFL IVVSTVLLFL RKQVRMLDVE KCGVEDTSSK NRNDTQFEET | 53 |
| Rhesus monkey PD-L1 (rmPD-L1) NP_001077 358.1 | MRIFAVFIFT IYWHLLNAFT VTVPKDLYVV EYGSNMTIEC RFPVEKQLGL TSLIVYWEME DKNIIQFVHG EEDLKVQHSN YRQRAQLLKD QLSLGNAALR ITDVKLQDAG VYRCMISYGG ADYKRITVKV NAPYNKINQR ILVVDPVTSE HELTCQAEGY PKAEVIWTSS DHQVLSGKTT TTNSKREEKL LNVTSTLRIN TTANEIFYCI FRRLGPEENH TAELVIPELP LALPPNERTH LVILGAIFLL LGVALTFIFY LRKGRMMDMK KSGIRVTNSK KQRDTQLEET | 54 |
| Chimeric PD-L1 (chiPD-L1) | MRIFAGIIFTACCHLLRAFTVTVPKDLYVVEYGSNMTIECKFP VEKQLDLAALIVYWEMEDKNIIQFVHGEEDLKVQHSSYRQRA RLLKDQLSLGNAALQITDVKLQDAGVYRCMISYGGADYKRIT VKVNAPYRKINQRISVDPATSEHELICQAEGYPEAEVIWTNSD HQPVSGKRSVTTSRTEGMLLNVTSSLRVNATANDVFYCTFWR SQPGQNHTAELIIPELPATHPPQNRTHWVLLGSILLFLIVVSTVL LFLRKQVRMLDVEKCGVEDTSSKNRNDTQFEET | 55 |

The test was detailed as follows: a 96-well cell culture plate was used, and the above-mentioned CHO cells expressing different PD-L1 proteins (25µl, 2x10⁴ cells/well) was added to each well, and then 25µl of the ascites purified anti-PD-L1 antibodies (1µg/ml) were added to each well. The mixture was incubated at 4°C for 30 minutes. The 96-well cell culture plate was centrifuged at 1200 rpm for 5 minutes and washed twice with PBS. Subsequently, 50 µl of anti-mouse IgG Fc-FITC at 1 : 100 dilution was added to each well, and was incubated at 4°C for 30 minutes. Subsequently, the 96-well cell culture plate was centrifuged at 1200 rpm for 5 minutes, and washed once with PBS. Then 200 µl of PBS was added to each well, and flow cytometry analysis was performed.

As shown in Figure 2A and Figure 2B, three ascites purified anti-PD-L1 antibodies (08-3F2, 07-6A11 and 17-7E4) and four anti-PD-L1 chimeric antibodies (24-1F4-mHvKv-IgG1, 33-10C9-mHvKv-IgG1, 23-2A6-mHvKv-IgG1, 23-4A8-mHvKv-IgG1) did not cross react with the murine PD-L1 protein, but had a strong cross reactivity with the rhesus monkey PD-L1 protein and human-mouse chimeric PD-L1 protein.

### Binding affinity testing of antibodies

Subsequently, the binding affinity of the anti-PD-L1 chimeric antibody, humanized antibody, and marketed control antibody to hPD-L1-His (manufactured by Edison) were determined by surface plasmon resonance (Biacore T200 biosensor, Biacore, INC, Piscataway N.J.) equipped with pre-immobilized Protein A sensor chips.

The test was detailed as follows: the anti-PD-L1 chimeric antibodies (07-6A11-mHvKv-IgG1, 17-7E4-mHvKv-IgG1, 08-3F2-mHvKv-IgG1, 23-2A6-mHvKv-IgG1-N297A, 23-4A8-mHvKv-IgG1-N297A, 24-1F4-mHvKv-IgG1-N297A, 33-10C9-mHvKv-IgG1-N297A, 0.5 ug/ml), marketed antibodies (Atezolizumab, Avelumab, Duralumab, 0.5 ug/ml) used as control antibodies, and humanized antibodies (6A11-H1K3-IgG1, 6A11-H1K4-IgG1, 6A11-H2K3-IgG1, 6A11-H2K4-IgG1, 6A11-H3K3-IgG1, 6A11-H3K4-IgG1, 6A11-H4K3-IgG1, 6A11-H4K4-IgG1, 7E4-H1K1-IgG1, 7E4-H1K2-IgG1, 7E4-H1K3-IgG1, 7E4-H2K1-IgG1, 7E4-H2K2-IgG1, 7E4-H2K3-IgG1, 7E4-H3K1-IgG1, 7E4-H3K2-IgG1, 7E4-H3K3- IgG1, 3F2-H1K1-IgG1, 3F2-H1K2-IgG1, 3F2-H1K3-IgG1, 3F2-H2K1-IgG1, 3F2-H2K2-IgG1, 3F2-H2K3-IgG1, 3F2-H3K1-IgG1, 3F2-H3K2-IgG1 3F2-H3K3-IgG1, 3F2-H4K1-IgG1, 3F2-H4K2-IgG1 and 3F2-H4K3-IgG1) were injected into the sensor chip (10 µl/min, 25 s) to achieve to a protein density at about 45-65RU. Then the hPD-L1-His proteins were injected into the sensor chip (30 µl/min, 100-400 s) at a concentration of 200nM, 100nM, 50nM, 25nM, 12.5nM, 6.25nM, 3.125nM, 1.5625 nM or 0.78125 nM, and the dissociation was monitored for 300 -1000 seconds. The detection results were read after the chip was regenerated with pH 2.0 glycine (30 µl/min, 10-20s). Kinetic association rates (kon) and dissociation rates (koff) were obtained simultaneously by fitting the data globally to a 1 : 1 Langmuir binding model (Karlsson, R. Roos, H. Fagerstam, L. Petersson, B., 1994. Methods Enzymology 6. 99-110) using Biacore T200 Evaluation Software 3.0. Affinities were deduced from the quotient of the kinetic rate constants (KD=koff/kon). The results for the tested antibodies are summarized in Table 6.

**Table 6. Binding Affinity of the anti-PD-L1 antibodies**

| **Anti-PD-L1 antibody** | | **Association rate kon (1/Ms)** | **Dissociation rate koff (1/s)** | **Affinity KD (M)** |
|---|---|---|---|---|
| Chimeric antibody | 07-6A11-mHvKv-IgG1 | 2.519E + 05 | 9.004E-04 | 3.574E-09 |
| | 17-7E4-mHvKv-IgGl | 1.510E + 05 | 8.048E-04 | 5.331E-09 |
| | 08-3F2-mHvKv-IgG1 | 1.621E + 06 | 4.304E-04 | 2.656E-10 |
| | 23-2A6-mHvKv-IgG1-N297A | 5.73E + 05 | 2.66E-03 | 4.64E-09 |
| | 23-4A8-mHvKv-IgG1-N297A | 3.61E + 05 | 5.32E-04 | 1.47E-09 |
| | 24-1F4-mHvKv-IgG1-N297A | 4.61E + 05 | 2.32E-04 | 5.04E-10 |
| | 33-10C9-mHvKv-IgG1-N297A | 1.22E + 05 | 1.96E-04 | 1.60E-09 |
| Control antibody | Atezolizumab | 1.144E + 05 | 2.710E-04 | 2.369E-09 |
| | Avelumab | 2.047E + 04 | 5.928E-05 | 2.896E-09 |
| | Durvalumab | 9.891E + 05 | 2.057E-04 | 2.079E-10 |
| 6A11 humanized antibody | 6A11-H1K3-IgG1 | 1.45E + 05 | 1.13E-03 | 7.80E-09 |
| | 6A11-H1K4-IgG1 | 1.49E + 05 | 1.24E-03 | 8.29E-09 |
| | 6A11-H2K3-IgG1 | 1.62E + 05 | 1.30E-03 | 8.07E-09 |
| | 6A11-H2K4-IgG1 | 2.18E + 05 | 1.53E-03 | 7.01E-09 |
| | 6A11-H3K3-IgG1 | 2.65E + 05 | 1.30E-03 | 4.91E-09 |
| | 6A11-H3K4-IgG1 | 2.58E + 05 | 1.68E-03 | 6.52E-09 |
| | 6A11-H4K3-IgG1 | 2.33E + 05 | 1.65E-03 | 7.09E-09 |
| | 6A11-H4K4-IgG1 | 2.08E + 05 | 2.00E-03 | 9.59E-09 |
| 7E4 humanized antibody | 7E4-H1K1-IgG1 | 1.18E + 05 | 3.13E-03 | 2.65E-08 |
| | 7E4-H1K2-IgG1 | 1.26E + 05 | 2.50E-03 | 1.98E-08 |
| | 7E4-H1K3-IgG1 | 1.28E + 05 | 1.70E-03 | 1.33E-08 |
| | 7E4-H2K1-IgG1 | 1.28E + 05 | 3.44E-03 | 2.70E-08 |
| | 7E4-H2K2-IgG1 | 1.23E + 05 | 2.70E-03 | 2.19E-08 |
| | 7E4-H2K3-IgG1 | 1.28E + 05 | 1.63E-03 | 1.27E-08 |
| | 7E4-H3K1-IgG1 | 1.47E + 05 | 3.52E-03 | 2.39E-08 |
| | 7E4-H3K2-IgG1 | 1.55E + 05 | 2.99E-03 | 1.93E-08 |
| | 7E4-H3K3-IgG1 | 1.50E + 05 | 2.18E-03 | 1.45E-08 |
| 3F2 humanized antibody | 3F2-HIK1-IgG1 | 9.81E + 05 | 7. 72E-04 | 7.87E-10 |
| | 3F2-H1K2-IgG1 | 1.06E + 06 | 7.32E-04 | 6.90E-10 |
| | 3F2-H1K3-IgG1 | 1.09E + 06 | 8.74E-04 | 8.05E-10 |
| | 3F2-H2K1-IgG1 | 1.13E + 06 | 7.92E-04 | 7.00E-10 |
| | 3F2-H2K2-IgG1 | 1.17E + 06 | 9.46E-04 | 8.10E-10 |
| | 3F2-H2K3-IgG1 | 1.16E + 06 | 8.81E-04 | 7.58E-10 |
| | 3F2-H3K1-IgG1 | 1.13E + 06 | 7.93E-04 | 6.99E-10 |
| | 3F2-H3K2-IgG1 | 1.21E + 06 | 7.53E-04 | 6.23E-10 |
| | 3F2-H3K3-IgG1 | 1.32E + 06 | 8.27E-04 | 6.25E-10 |
| | 3F2-H4K1-IgG1 | 1.33E + 06 | 6.26E-04 | 4.71E-10 |
| | 3F2-H4K2-IgG1 | 1.30E + 06 | 6.17E-04 | 4.75E-10 |
| | 3F2-H4K3-IgG1 | 1.39E + 06 | 6.96E-04 | 5.02E-10 |

### Example 4. Thermal stability of antibodies

Thermofluor assay analysis was performed using Protein Thermal Shift Dye Kit (Thermo Fisher Scientific) and QuantStudio^{™} 5 Real Time PCR Systems (Thermo Fisher Scientific). This assay measured thermostability using a fluorescent dye that binds to hydrophobic patches exposed as the protein unfolds after heating. The experiments were performed according to the manufacturer's protocol" 2µl of the antibody, 10.5µl of water, 5µl of Protein Thermal Shift buffer and 25µl of Protein Thermal Shift Dye were mixed, and heated to 25°C at 1.6°C/second, and then heated to 99°C at 0.05°C/second. The denaturation temperature Tm value of the antibody was tested (if there were two transition peaks, the second denaturation of the Fab domains was treated as Tm).

Table 7 showed the Tm values of the chimeric antibodies and humanized antibodies. Further to reduce antibody-dependent cell-mediated cytotoxicity (ADCC) and complement-dependent cytotoxicity (CDC), antibodies were engineered to reduce glycan heterogeneity to improve the efficacy and safety. Some amino acids in Fc regions of the antibody were replaced, for example, N297A mutation.

**Table 7. Thermal stability of the antibodies**

| **Antibody type** | **Antibody name** | **Tm value (°C)** |
|---|---|---|
| 3F2 chimeric **antibody** | 08-3F2-mHvKv-IgG1 | 74.46 |
| 3F2 humanized antibody | 3F2-H1K1-IgG1 | 79.71 |
| | 3F2-H1K2-IgG1 | 80.00 |
| | 3F2-H1K3-IgG1 | 78.38 |
| | 3F2-H2K1-IgG1 | 71.87 |
| | 3F2-H2K2-IgG1 | 71.80 |
| | 3F2-H2K3-IgG1 | 70.47 |
| | 3F2-H3K1-IgG1 | 72.31 |
| | 3F2-H3K2-IgG1 | 72.54 |
| | 3F2-H3K3-IgG1 | 71.21 |
| | 3F2-H4K1-IgG1 | 75.86 |
| | 3F2-H4K2-IgG1 | 76.01 |
| | 3F2-H4K3-IgG1 | 74.46 |
| 7E4 chimeric antibody | 17-7E4-mHvKv-IgG1 | 74.09 |
| 7E4 humanized antibody | 7E4-H1K1-IgG1 | 81.78 |
| | 7E4-H1K2-IgG1 | 82.82 |
| | 7E4-H1K3-IgG1 | 84.3 |
| | 7E4-H2K1-IgG1 | 82.08 |
| | 7E4-H2K2-IgG1 | 82.89 |
| | 7E4-H2K3-IgG1 | 83.41 |
| | 7E4-H3K1-IgG1 | 82.59 |
| | 7E4-H3K2-IgG1 | 83.11 |
| | 7E4-H3K3-IgG1 | 83.63 |
| 6A11 chimeric antibody | 07-6A11-mHvKv-IgG1 | 75.53 |
| | 07-6A11-mHvKv-IgG4 | 76.09 |
| | 07-6A11-mHvKv-IgG1-N297A | 76.87 |
| 6A11 humanized antibody | 6A11-H1K1-IgG1 | 78.05 |
| | 6A11-H1K3-IgG1 | 67.25 |
| | 6A11-H1K4-IgG1 | 70.21 |
| | 6A11-H2K1-IgG1 | 76.87 |
| | 6A11-H2K3-IgG1 | 64.81 |
| | 6A11-H2K4-IgG1 | 68.80 |
| | 6A11-H3K1-IgG1 | 77.01 |
| | 6A11-H3K3-IgG1 | 65.10 |
| | 6A11-H3K4-IgG1 | 68.80 |
| | 6A11-H4K1-IgG1 | 75.98 |
| | 6A11-H4K3-IgG1 | 63.25 |
| | 6A11-H4K4-IgG1 | 67.17 |

### Example 5. In vivo efficacy testing of antibodies

In order to detect the *in vivo* efficacy of anti-PD-L1 antibodies, humanized mice with PD-L1 gene were used to generate tumor animal models. The mouse express the human-mouse chimeric PD-L1 protein (SEQ ID NO: 55), wherein the extracellular region of the mouse PD-L1 protein was replaced by a human sequence: amino acids 21-128 of the mouse PD-L1 protein (SEQ ID NO: 53) was replaced by amino acids 21-128 of human PD-L1 protein (SEQ ID NO: 52). The B-hPD-L1 mouse model provided a new tool for preclinical animal experiments of PD-L1 monoclonal antibody testing, by which significantly improved the predictability of clinical trials (see PCT application number PCT/CN 2017/099574 and Chinese application number 201710757022.6, which are incorporated herein by reference in their entirety).

The tumor animal model was prepared as follows: B-hPD-L1 mice were inoculated with mouse MC-38 cells (colon cancer cells) with humanized PD-L1 gene by subcutaneous injection. After the tumor volume reached 150 ± 50mm³, the mice were randomly divided into an anti-PD-L1 antibody treatment group and control group (physiological saline), and administered by intraperitoneal injection. The body weight and tumor volume of the mice were measured regularly twice a week. Tumor volume (mm³) = 0.5 x long diameter x short diameter². The injected volume (0.3 mg/kg or 3 mg/kg) was calculated based on the body weight of the mouse.

The tumor growth inhibition percentage (TGI) was calculated using the following formula: TGI (%) = [1-(Ti-T0)/(Vi-V0)] × 100, where Ti is the average tumor volume on day i in the treatment group; T0 is the average tumor volume on day 0 of the treatment group; Vi is the average tumor volume on day i of the control group; and V0 is the average tumor volume on day 0 of the control group. T-test was performed for statistical analysis. A TGI% higher than 60% indicates significant suppression of tumor growth. P < 0.05 is a threshold to indicate significant difference.

### In vivo efficacy result of anti-PD-L1 murine antibodies 08-3F2, 17-7E4, 17-8E9 and 07-6A11

A total of thirty B-hPD-L1 mice (5-6 weeks) were subcutaneously injected with MC38-hPD-L1 cells (5 x 10⁵/mouse). When the tumor reached a volume of 150 ± 50 mm³, the mice were randomly placed into 7 groups, with 5 mice in each group. The treatment groups were treated with anti-PD-L1 antibodies 08-3F2, 17-7E4, 17-8E9, 07-6A11 or the positive control antibody Atezolizumab respectively by intraperitoneal injection at a dose of 3 mg/kg, and the control group was injected with physiological saline, and administered on day 1, 3, and 5 of each week. The body weight and tumor volume of the mice were measured twice a week until the end of the experiment after 3 weeks. As shown in Figure 3A and Figure 3B, the average body weight of the mice in the control group and the treatment groups increased steadily during the entire treatment period, and there was no significant difference between the groups, indicating that these anti-PD-L1 antibodies were not obviously toxic to the mice. As shown in Figure 4 (tumor volume data,21 days after grouping), compared with the control group, the tumor growth in the treatment groups were inhibited to different extents. Table 8 below showed the TGI% results for each group.

**Table 8. Tumor growth inhibition rate**

| **Group** | **Antibody** | **Average tumor volume (mm³)** | **TGI%** | **P value** |
|---|---|---|---|---|
| G1 | Physiological saline (PS) | 2143 ± 410 | - | - |
| G2 | 08-3F2 (3 mg/kg) | 264 ± 82 | 94.2% | 0.002 |
| G3 | 17-7E4 (3 mg/kg) | 544 ± 232 | 80.2% | 0.009 |
| G4 | 17-8E9 (3 mg/kg) | 1017 ± 302 | 56.4% | 0.058 |
| G5 | 07-6A11 (3 mg/kg) | 345 ± 114 | 90.1% | 0.003 |
| G6 | Atezolizumab (3 mg/kg) | 627±216 | 76.0% | 0.011 |

The above results indicate that the anti-PD-L1 murine antibodies of the present invention 08-3F2, 07-6A11, 17-7E4 and 17-8E9 exhibited tumor inhibitory effects, wherein the 08-3F2, 07-6A11 and 17-7E4 treatment groups obtained better tumor inhibitory effect as compared to that of positive control antibody Atezolizumab.

### In vivo efficacy testing of chimeric antibodies 07-6A11-mHvKv-IgG1, 07-6A11-mHvKv-IgG4, and 07-6A11-mHvKv-IgG1-N297A

A total of thirty B-hPD-L1 mice (5-6 weeks) were subcutaneously injected with MC38-hPD-L1 cells (5x10⁵/mouse). When the tumor reached a volume of 150±50mm³, the mice were randomly placed into 7 groups, with 5 mice in each group. The treatment groups were treated with anti-PD-L1 chimeric antibodies 07-6A11-mHvKv-IgG1, 07-6A11-mHvKv-IgG4, 07-6A11-mHvKv-IgG1-N297A, or murine antibody 07-6A11 respectively by intraperitoneal injection, and the control group was treated with hIgG antibody at a dose of 3 mg/kg, and administered on day 1, 3, and 5 of the week. The body weight and tumor volume of the mice were measured twice a week until the end of the experiment after 3 weeks. The average body weight of the mice in the control group and the treatment groups increased steadily during the entire treatment, and there was no significant difference between the groups, indicating that these anti-PD-L1 antibodies were not obviously toxic to the mice. In addition, compared with the control group, the tumor growth in the treatment groups were inhibited to different extents.

### In vivo efficacy testing of chimeric antibodies 08-3F2-mHvKv-IgG4, 08-3F2-mHvKv-IgG1-N297A, 17-7E4-mHvKv-IgG4 and 17-7E4-mHvKv-IgG1-N297A

A total of thirty-five B-hPD-L1 mice (5-6 weeks) were subcutaneously injected with MC38-hPD-L1 cells (5 x 10⁵/mouse). When the tumor reached a volume of 150 ± 50 mm³, the mice were randomly placed into 7 groups, with 5 mice in each group. The treatment groups were treated with anti-PD-L1 chimeric antibodies 08-3F2-mHvKv-IgG4, 08-3F2-mHvKv-IgG1-N297A, 17-7E4-mHvKv-IgG4, 17-7E4-mHvKv-IgG1-N297A, or murine antibodies 07-6A11 or 08-3F2 respectively by intraperitoneal injection at a dose of 3 mg/kg, and the control group was injected with physiological saline, and administered on day 1, 3, and 5 of the week. The body weight and tumor volume of the mice were measured twice a week, and until the end of the experiment after 3 weeks. The average body weight of the mice in the control group and the treatment groups increased steadily during the entire treatment, and there was no significant difference between the groups, indicating that these anti-PD-L1 antibodies were not obviously toxic to the mice. In addition, compared with the control group, the tumor growth in the treatment groups were inhibited to different extents.

### In vivo efficacy testing of chimeric antibodies 33-10C9-mHvKv-IgG1 24-1F4-mHvKv-IgG1 23-2A6-mHvKv-IgG1, 23-4A8-mHvKv-IgG1 23-2A5-mHvKv-IgG1 and 24-1C3-mHvKv-IgG1

A total of thirty-five B-hPD-L1 mice (5-6 weeks) were subcutaneously injected with MC38-hPD-L1 cells (5 x 10⁵/mouse). When the tumor reached a volume of 150 ± 50 mm³, the mice were randomly placed into 7 groups, with 5 mice in each group. The treatment groups were treated with anti-PD-L1 chimeric antibodies 33-10C9-mHvKv-IgG1, 24-1F4-mHvKv-IgG1, 23-2A6-mHvKv-IgG1, 23-4A8-mHvKv-IgG1, 23-2A5-mHvKv-IgG1 or 24-1C3-mHvKv-IgG1 respectively by intraperitoneal injection at a dose of 3 mg/kg, and the control group was injected with physiological saline, and administered on day 1, 3, and 5 of the week. The body weight and tumor volume of the mice were measured twice a week until the end of the experiment after 3 weeks. As shown in Figure 5A and Figure 5B, the average body weight of the mice in the control group and the treatment groups increased steadily during the entire treatment, and there was no significant difference between the groups, indicating that these anti-PD-L1 antibodies were not obviously toxic to the mice. As shown in Figure 6 (tumor volume data, 25 days after grouping), compared with the control group, the tumor growth in the treatment groups were inhibited to different extents. Among them, the chimeric antibody 23-2A6-mHvKv-IgG1 exhibited the best inhibitory effect, followed by 33-10C9-mHvKv-IgG1 and 23-4A8-mHvKv-IgG1. Table 9 below showed the TGI% results for each group.

**Table 9. Tumor growth inhibition rate**

| **Group** | **Antibody** | **Average tumor volume (mm³)** | **TGI%** | **P value** |
|---|---|---|---|---|
| G1 | Physiological saline (PS) | 1878 ± 625 | - | - |
| G2 | 33-10C9-mHvKv-IgG1 (3 mg/kg) | 932 ± 221 | 50.4% | 0.191 |
| G3 | 24-IF4-mHvKv-IgG1 (3 mg/kg) | 986 ± 271 | 47.5% | 0.226 |
| G4 | 23-2A6-mHvKv-IgG1 (3 mg/kg) | 745 ± 151 | 60.3% | 0.116 |
| G5 | 23-4A8-mHvKv-IgG1 (3 mg/kg) | 937 ± 314 | 50.1% | 0.215 |
| G6 | 23-2A5-mHvKv-IgG1 (3 mg/kg) | 1139 ± 145 | 39.4% | 0.282 |
| G7 | 24-1C3-mHvKv-IgG1 (3 mg/kg) | 1211 ± 193 | 35.5% | 0.337 |

### In vivo efficacy testing of humanized antibodies 6A11-H1K3-IgG1-N297A, 6A11-H1K4-IgG1-N297A, 3F2-H1K1-IgG1-N297A, 3F2-H1K2-IgG1-N297A, 7E4-H1K1-IgG1-N297A and 7E4-H2K1-IgG1-N297A

A total of fifty-six B-hPD-L1 mice (5-6 weeks) were subcutaneously injected with MC38-hPD-L1 cells (5 x 10⁵/mouse). When the tumor reached a volume of 150 ± 50 mm³, the mice were randomly placed into 7 groups, with 8 mice in each group. The treatment groups were treated with anti-PD-L1 humanized antibodies 6A11-H1K3-IgG1-N297A, 6A11-H1K4-IgG1-N297A, 3F2-H1K1-IgG1-N297A, 3F2-H1K2-IgG1-N297A, 7E4-H1K1-IgG1-N297A or 7E4-H2K1-IgG1-N297A respectively by intraperitoneal injection at a dose of 3 mg/kg, and the control group was injected with physiological saline, and administered on day 2 and 5 of the week. The body weight and tumor volume of the mice were measured twice a week until the end of the experiment after 3 weeks. As shown in Figure 7A and Figure 7B, the average body weight of the mice in the control group and the treatment groups increased steadily during the entire treatment period, and there was no significant difference between the groups, indicating that these anti-PD-L1 antibodies were not obviously toxic to the mice. As shown in Figure 8 (tumor volume data, 24 days after grouping), compared with the control group, the tumor growth in the treatment groups were inhibited to different extents. Among them, the humanized antibody 6A11-H1K3-IgG1-N297A exhibited the best inhibitory effect, followed by 3F2-H1K2-IgG1-N297A. Table 12 below showed the TGI% results for each group.

**Table 10. Tumor growth inhibition rate**

| **Group** | **Antibody** | **Average tumor volume (mm³)** | **TGI%** | **P value** |
|---|---|---|---|---|
| G1 | Physiological saline (PS) | 2133 ± 210 | - | - |
| G2 | 6A11-H1K3-IgG1-N297A (3 mg/kg) | 1009 ± 225 | 55.7% | 0.003 |
| G3 | 6A11-H1K4-IgG1-N297A (3 mg/kg) | 1154 ± 279 | 48.6% | 0.014 |
| G4 | 3F2-HlKl-IgGl-N297A (3 mg/kg) | 1298 ± 213 | 41.4% | 0.014 |
| G5 | 3F2-HlK2-IgGl-N297A (3 mg/kg) | 1124 ± 242 | 50.0% | 0.007 |
| G6 | 7E4-H1K1-IgG1-N297A (3 mg/kg) | 1530 ± 287 | 29.8% | 0.112 |
| G7 | 7E4-H2K1-IgG1-N297A (3 mg/kg) | 1137 ± 213 | 49.4% | 0.005 |

### In vivo efficacy testing at different dose level of the anti-PD-L1 antibody

A total of twenty B-hPD-L1 mice (5-6 weeks) were subcutaneously injected with MC38-hPD-L1 cells (5 x 10⁵/mouse). When the tumor reached a volume of 150 ± 50 mm³, the mice were randomly placed into 4 groups, with 5 mice in each group. The treatment groups were treated with 1 mg/kg, 3 mg/kg or 10 mg/kg anti-PD-L1 antibody 07-6A11 respectively by intraperitoneal injection, and the control group was injected with physiological saline, and administered once every two days for 8 times of administrations in total. The body weight and tumor volume of the mice were measured twice a week until the end of the experiment after 3 weeks. As shown in Figure 9A and Figure 9B, the average body weight of the mice in the control group and the treatment groups increased steadily during the entire treatment period, and there was no significant difference between the groups, indicating that these anti-PD-L1 antibodies were not obviously toxic to the mice. As shown in Figure 10 (tumor volume data, 18 days after grouping), compared with the control group, the tumor growth in the treatment groups were inhibited to different extents, and the larger the antibody dose, the better the tumor inhibition effect. Table 13 below showed the TGI% results for each group.

**Table 11. Tumor growth inhibition rate**

| **Grouping** | **Antibody** | **Average tumor volume (mm³)** | **TGI%** | **P value** |
|---|---|---|---|---|
| G1 | Physiological saline | 1200 ± 193 | - | - |
| G2 | 07-6A11 (10 mg/kg) | 111 ± 66 | 101.2% | 0.001 |
| G3 | 07-6A11 (3 mg/kg) | 251 ± 87 | 88.2% | 0.002 |
| G4 | 07-6A11 (1 mg/kg) | 448 ± 253 | 69.9% | 0.046 |

### In vivo efficacy test of anti-PD-L1 antibody in combination with additional therapeutic agents

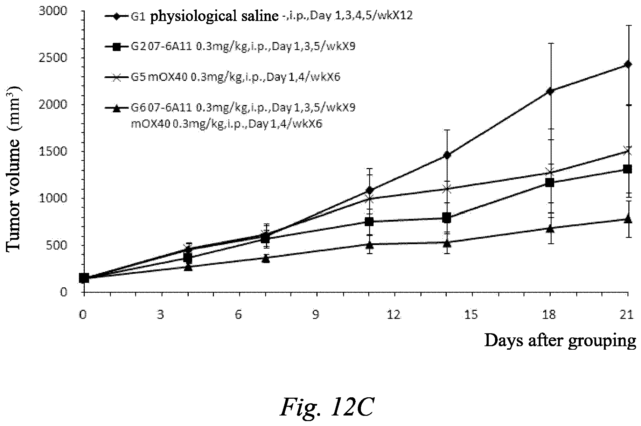

A total of thirty-five B-hPD-L1 mice (5-6 weeks) were subcutaneously injected with MC38-hPD-L1 cells (5 x 10⁵/mouse). When the tumor reached a volume of 150 ± 50 mm³, the mice were randomly placed into 7 groups, with 5 mice in each group. The treatment groups were treated with the anti-PD-L1 murine antibody 07-6A11 (0.3 mg/kg), anti-CTLA4 antibody mCTLA4 (1 mg/kg), anti-OX40 antibody mOX40 (0.3 mg/kg) or combination thereof respectively by intraperitoneal injection, and the control group was injected with physiological saline. Among them, the anti-PD-L1 antibody was administered on day 1, 3, and 5 of each week, and the anti-CTLA4 antibody and anti-OX40 were administered on days 1 and 4 of each week. The body weight and tumor volume of the mice were measured twice a week until the end of the experiment after 3 weeks. As shown in Figure 11A and Figure 11B, the average body weight of the mice in the control group and the treatment group increased steadily during the entire treatment period, and there was no significant difference between the groups, indicating that the above three antibodies were not obviously toxic to the mice. As shown in Figure 12A, Figure 12B and Figure 12C (tumor volume data, 21 days after grouping), compared with the control group, the tumor growth in the treatment groups were inhibited to different extents. Table 14 below showed the TGI% results for each group. The results showed that the anti-PD-L1 antibody in combination with anti-CTLA4 antibody, or anti-OX40 antibody can significantly inhibit tumor growth with superior efficacy. Among them, the mCTLA4 antibody was purchased from BioXcell with a catalog number of BE0164; and mOX40 antibody was purchased from BioXcell with a catalog number of BE0031.

**Table 12. Tumor growth inhibition rate**

| **Group** | **Antibody** | **Average tumor volume (mm³)** | **TGI%** | **P value** |
|---|---|---|---|---|
| G1 | Physiological saline | 2430 ± 422 | - | - |
| G2 | 07-6A11 (0.3 mg/kg) | 1307 ± 248 | 49.2% | 0.051 |
| G3 | mCTLA-4 (1 mg/kg) | 2234 ± 598 | 8.6% | 0.796 |
| G4 | 07-6A11 (0.3 mg/kg) mCTLA-4 (1 mg/kg) | 840 ± 309 | 69.7% | 0.016 |
| G5 | mOX40 (0.3 mg/kg) | 1505 ± 492 | 40.6% | 0.192 |
| G6 | 07-6A11 (0.3 mg/kg) mOX40 (0.3 mg/kg) | 783 ± 194 | 72.3% | 0.008 |

## Claims

1. An anti-PD-L1 antibody or antigen binding fragment thereof, wherein the antibody comprises a heavy chain complementarity determining region (CDR H) comprising:
CDR H1 comprising an amino acid sequence selected from SEQ ID NO: 7, SEQ ID NO: 13, SEQ ID NO: 19, SEQ ID NO: 64, SEQ ID NO: 70, SEQ ID NO: 76 or SEQ ID NO: 82;
CDR H2 comprising an amino acid sequence selected from SEQ ID NO: 8, SEQ ID NO: 14, SEQ ID NO: 20, SEQ ID NO: 65, SEQ ID NO: 71, SEQ ID NO: 77 or SEQ ID NO: 83; and
CDR H3 comprising an amino acid sequence selected from SEQ ID NO: 9, SEQ ID NO: 15, SEQ ID NO: 21, SEQ ID NO: 66, SEQ ID NO: 72, SEQ ID NO: 78 or SEQ ID NO: 84.

2. The anti-PD-L1 antibody or antigen binding fragment thereof of claim 1, wherein the antibody comprises the CDR H comprising:
a) CDR H1, CDR H2 and CDR H3 comprising SEQ ID NO: 7, SEQ ID NO: 8 and SEQ ID NO: 9, respectively;
b) CDR HI, CDR H2 and CDR H3 comprising SEQ ID NO: 13, SEQ ID NO: 14 and SEQ ID NO: 15, respectively;
c) CDR HI, CDR H2 and CDR H3 comprising SEQ ID NO: 19, SEQ ID NO: 20 and SEQ ID NO: 21, respectively;
d) CDR HI, CDR H2 and CDR H3 comprising SEQ ID NO: 64, SEQ ID NO: 65 and SEQ ID NO: 66, respectively;
e) CDR HI, CDR H2 and CDR H3 comprising SEQ ID NO: 70, SEQ ID NO: 71 and SEQ ID NO: 72, respectively;
f) CDR H1, CDR H2 and CDR H3 comprising SEQ ID NO: 76, SEQ ID NO: 77 and SEQ ID NO: 78, respectively; or
g) CDR H1, CDR H2 and CDR H3 comprising SEQ ID NO: 82, SEQ ID NO: 83 and SEQ ID NO: 84, respectively.

3. The anti-PD-L1 antibody or antigen binding fragment thereof of claim 1 or 2, wherein the antibody further comprises a light chain complementarity determining regions (CDR L) comprising:
CDR L1 comprising an amino acid sequence selected from SEQ ID NO: 10, SEQ ID NO: 16, SEQ ID NO: 22, SEQ ID NO: 67, SEQ ID NO: 73, SEQ ID NO: 79 or SEQ ID NO: 85;
CDR L2 comprising an amino acid sequence selected from SEQ ID NO: 11, SEQ ID NO: 17, SEQ ID NO: 23, SEQ ID NO: 68, SEQ ID NO: 74, SEQ ID NO: 80 or SEQ ID NO: 86; and
CDR L3 comprising an amino acid sequence selected from SEQ ID NO: 12, SEQ ID NO: 18, SEQ ID NO: 24, SEQ ID NO: 69, SEQ ID NO: 75, SEQ ID NO: 81 or SEQ ID NO: 87.

4. The anti-PD-L1 antibody or antigen binding fragment thereof of claim 3, wherein the antibody comprises the CDR L comprising:
a) CDR L1, CDR L2 and CDR L3 comprising SEQ ID NO: 10, SEQ ID NO: 11 and SEQ ID NO: 12, respectively;
b) CDR L1, CDR L2 and CDR L3 comprising SEQ ID NO: 16, SEQ ID NO: 17 and SEQ ID NO: 18, respectively;
c) CDR L1, CDR L2 and CDR L3 comprising SEQ ID NO: 22, SEQ ID NO: 23 and SEQ ID NO: 24, respectively;
d) CDR L1, CDR L2 and CDR L3 comprising SEQ ID NO: 67, SEQ ID NO: 68 and SEQ ID NO: 69, respectively;
e) CDR L1, CDR L2 and CDR L3 comprising SEQ ID NO: 73, SEQ ID NO: 74 and SEQ ID NO: 75, respectively;
f) CDR L1, CDR L2 and CDR L3 comprising SEQ ID NO: 79, SEQ ID NO: 80 and SEQ ID NO: 81, respectively; or
g) CDR L1, CDR L2 and CDR L3 comprising SEQ ID NO: 85, SEQ ID NO: 86 and SEQ ID NO: 87, respectively.

5. The anti-PD-L1 antibody or antigen binding fragment thereof of claim 4, wherein the antibody comprises a CDR H and CDR L comprising:
a) CDR H1, CDR H2 and CDR H3 comprising SEQ ID NO: 7, SEQ ID NO: 8 and SEQ ID NO: 9, respectively, and CDR L1, CDR L2 and CDR L3 comprising SEQ ID NO: 10, SEQ ID NO: 11 and SEQ ID NO: 12, respectively;
b) CDR H1, CDR H2 and CDR H3 comprising SEQ ID NO: 13, SEQ ID NO: 14 and SEQ ID NO: 15, respectively, and CDR L1, CDR L2 and CDR L3 comprising SEQ ID NO: 16, SEQ ID NO: 17 and SEQ ID NO: 18, respectively;
c) CDR H1, CDR H2 and CDR H3 comprising SEQ ID NO: 19, SEQ ID NO: 20 and SEQ ID NO: 21, respectively, and CDR L1, CDR L2 and CDR L3 comprising SEQ ID NO: 22, SEQ ID NO: 23 and SEQ ID NO: 24, respectively;
d) CDR H1, CDR H2 and CDR H3 comprising SEQ ID NO: 64, SEQ ID NO: 65 and SEQ ID NO: 66, respectively, and CDR L1, CDR L2 and CDR L3 comprising SEQ ID NO: 67, SEQ ID NO: 68 and SEQ ID NO: 69, respectively;
e) CDR H1, CDR H2 and CDR H3 comprising SEQ ID NO: 70, SEQ ID NO: 71 and SEQ ID NO: 72, respectively, and CDR L1, CDR L2 and CDR L3 comprising SEQ ID NO: 73, SEQ ID NO: 74 and SEQ ID NO: 75, respectively;
f) CDR H1, CDR H2 and CDR H3 comprising SEQ ID NO: 76, SEQ ID NO: 77 and SEQ ID NO: 78, respectively, and CDR L1, CDR L2 and CDR L3 comprising SEQ ID NO: 79, SEQ ID NO: 80 and SEQ ID NO: 81, respectively; or
g) CDR H1, CDR H2 and CDR H3 comprising SEQ ID NO: 82, SEQ ID NO: 83 and SEQ ID NO: 84, respectively, and CDR L1, CDR L2 and CDR L3 comprising SEQ ID NO: 85, SEQ ID NO: 86 and SEQ ID NO: 87, respectively.

6. An anti-PD-L1 antibody or antigen binding fragment thereof, wherein the antibody comprises a heavy chain variable region (VH) comprising:
a) an amino acid sequence selected from SEQ ID NO: 1, SEQ ID NO: 3, SEQ ID NO: 5, SEQ ID NO: 56, SEQ ID NO: 58, SEQ ID NO: 60 or SEQ ID NO: 62;
b) an amino acid sequence that is at least 80%, such as at least 85%, at least 90%, at least 95%, at least 98%, or at least 99% identical to the amino acid sequence of a); or
c) an amino acid sequence with one or more amino acid modifications in the amino acid sequence of a).

7. The anti-PD-L1 antibody or antigen binding fragment thereof of claim 6, wherein the antibody comprises a light chain variable region (VL) comprising:
a) an amino acid sequence selected from SEQ ID NO: 2, SEQ ID NO: 4, SEQ ID NO: 6, SEQ ID NO: 57, SEQ ID NO: 59, SEQ ID NO: 61 or SEQ ID NO: 63;
b) an amino acid sequence that is at least 80%, such as at least 85%, at least 90%, at least 95%, at least 98%, or at least 99% identical to the amino acid sequence of a); or
c) an amino acid sequence with one or more amino acid modifications in the amino acid sequence of a).

8. The anti-PD-L1 antibody or antigen binding fragment thereof of claim 7, wherein the antibody comprises the following VH and VL:
a) VH and VL comprising the amino acid sequences of SEQ ID NO: 1 and SEQ ID NO: 2, respectively;
b) VH and VL comprising an amino acid sequence that is at least 80% , such as at least 85%, at least 90%, at least 95%, at least 98%, or at least 99% identical to SEQ ID NO: 1 and SEQ ID NO: 2, respectively; or
c) VH and VL comprising the amino acid sequences with one or more amino acid modifications in SEQ ID NO: 1 and SEQ ID NO: 2, respectively.

9. The anti-PD-L1 antibody or antigen binding fragment thereof of claim 7, wherein the antibody comprises the following VH and VL:
a) VH and VL comprising the amino acid sequences of SEQ ID NO: 3 and SEQ ID NO: 4 respectively;
b) VH and VL comprising an amino acid sequence that is at least 80% , such as at least 85%, at least 90%, at least 95%, at least 98%, or at least 99% identical to SEQ ID NO: 3 and SEQ ID NO: 4, respectively; or
c) VH and VL comprising the amino acid sequences with one or more amino acid modifications in SEQ ID NO: 3 and SEQ ID NO: 4, respectively.

10. The anti-PD-L1 antibody or antigen binding fragment thereof of claim 7, wherein the antibody comprises the following VH and VL:
a) VH and VL comprising the amino acid sequences of SEQ ID NO: 5 and SEQ ID NO: 6, respectively;
b) VH and VL comprising an amino acid sequence that is at least 80% , such as at least 85%, at least 90%, at least 95%, at least 98%, or at least 99% identical to SEQ ID NO: 5 and SEQ ID NO: 6; or
c) VH and VL comprising the amino acid sequences with one or more amino acid modifications in SEQ ID NO: 5 and SEQ ID NO: 6, respectively.

11. The anti-PD-L1 antibody or antigen binding fragment thereof of claim 7, wherein the antibody comprises the following VH and VL:
a) VH and VL comprising the amino acid sequences of SEQ ID NO: 56 and SEQ ID NO: 57, respectively;
b) VH and VL comprising an amino acid sequence that is at least 80% identical, such as at least 85%, at least 90%, at least 95%, at least 98%, or at least 99% identical to SEQ ID NO: 56 and SEQ ID NO: 57; or
c) VH and VL comprising the amino acid sequences with one or more amino acid modifications in SEQ ID NO: 56 and SEQ ID NO: 57, respectively.

12. The anti-PD-L1 antibody or antigen binding fragment thereof of claim 7, wherein the antibody comprises the following VH and VL:
a) VH and VL comprising the amino acid sequences of SEQ ID NO: 58 and SEQ ID NO: 59, respectively;
b) VH and VL comprising an amino acid sequence that is at least 80%, such as at least 85%, at least 90%, at least 95%, at least 98%, or at least 99% identical to SEQ ID NO: 58 and SEQ ID NO: 59; or
c) VH and VL comprising the amino acid sequences with one or more amino acid modifications in SEQ ID NO: 58 and SEQ ID NO: 59, respectively.

13. The anti-PD-L1 antibody or antigen binding fragment thereof of claim 7, wherein the antibody comprises the following VH and VL:
a) VH and VL comprising the amino acid sequences of SEQ ID NO: 60 and SEQ ID NO: 61, respectively;
b) VH and VL comprising an amino acid sequence that is at least 80%, such as at least 85%, at least 90%, at least 95%, at least 98%, or at least 99% identical to SEQ ID NO: 60 and SEQ ID NO: 61; or
c) VH and VL comprising the amino acid sequences with one or more amino acid modifications in SEQ ID NO: 60 and SEQ ID NO: 61, respectively.

14. The anti-PD-L1 antibody or antigen binding fragment thereof of claim 7, wherein the antibody comprises the following VH and VL:
a) VH and VL comprising the amino acid sequences of SEQ ID NO: 62 and SEQ ID NO: 63, respectively;
b) VH and VL comprising an amino acid sequence that is at least 80%, such as at least 85%, at least 90%, at least 95%, at least 98%, or at least 99% identical to SEQ ID NO: 62 and SEQ ID NO: 63; or
c) VH and VL comprising the amino acid sequences with one or more amino acid modifications in SEQ ID NO: 62 and SEQ ID NO: 63, respectively.

15. The anti-PD-L1 antibody or antigen binding fragment thereof of any one of claims 1-14, wherein the antibody is a murine antibody, a chimeric antibody, a humanized antibody, or a fully human antibody.

16. The anti-PD-L1 antibody or antigen binding fragment thereof of any one of claims 6-14, wherein the amino acid modification is located in a framework region of the variable region.

17. The anti-PD-L1 antibody or antigen binding fragment thereof of any one of claims 6-14, wherein the modification is humanization.

18. The anti-PD-L1 antibody or antigen binding fragment thereof of claim 8, wherein the antibody comprises VH comprising the amino acid sequence selected from SEQ ID NO: 39, SEQ ID NO: 40, SEQ ID NO: 41 or SEQ ID NO: 42, and VL comprising the amino acid sequence selected from SEQ ID NO: 43, SEQ ID NO: 44 or SEQ ID NO: 45.

19. The anti-PD-L1 antibody or antigen binding fragment thereof of claim 9, wherein the antibody comprises VH comprising the amino acid sequence selected from SEQ ID NO: 31, SEQ ID NO: 32, SEQ ID NO: 33 or SEQ ID NO: 34, and VL comprising the amino acid sequence selected from SEQ ID NO: 35, SEQ ID NO: 36, SEQ ID NO: 37 or SEQ ID NO: 38.

20. The anti-PD-L1 antibody or antigen binding fragment thereof of claim 10, wherein the antibody comprises VH comprising the amino acid sequence selected from SEQ ID NO: 46, SEQ ID NO: 47 or SEQ ID NO: 48, and VL comprising an amino acid sequence selected from SEQ ID NO: 49, SEQ ID NO: 50 or SEQ ID NO: 51.

21. The anti-PD-L1 antibody or antigen binding fragment thereof of any one of claims 1-20, wherein the antibody comprises an Fc region amino acid modification which reduces the antibody-dependent cell-mediated cytotoxicity (ADCC) and/or complement-dependent cytotoxicity (CDC) of the antibody.

22. The anti-PD-L1 antibody or antigen binding fragment thereof of claim 21, wherein the modification comprises an N297A mutation.

23. The anti-PD-L1 antibody or antigen binding fragment thereof of any one of claims 1-22, wherein the antibody is selected from an IgG, an IgA, an IgM, an IgE or an IgD isotype.

24. The anti-PD-L1 antibody or antigen binding fragment thereof of any one of claims 1-23, wherein the antibody is selected from an IgG1, an IgG2, an IgG3 or an IgG4 subtype.

25. The anti-PD-L1 antibody or antigen binding fragment thereof of any one of claims 1-24, wherein the antigen binding fragment is selected from a Fab fragment, a Fab' fragment, a Fd fragment, a Fd' fragment, a Fv fragment, a dAb Fragment, a F(ab')₂ fragment, a single chain fragment, a diabody or a linear antibody.

26. A bispecific antibody comprising a first antigen binding region that binds to PD-L1, and a second antigen binding region that binds to a second antigen, wherein the first antigen binding region comprises the CDR H1,CDR H2 and CDR H3 and/or CDR L1, CDR L2 and CDR L3 of the anti-PD-L1 antibody of any one of claims 1-5, or the VH and/or VL of the anti-PD-L1 antibody of any one of claims 6-20.

27. The bispecific antibody of claim 26, wherein the second antigen is selected from a tumor-associated antigen or an immune checkpoint molecule.

28. A nucleotide sequence encoding a polypeptide of the anti-PD-L 1 antibody of any one of claims 1-25.

29. A nucleotide sequence encoding a VH or VL of the anti-PD-L1 antibody of any one of claims 6-20.

30. A vector comprising the nucleotide sequence of claim 28 or 29.

31. A recombinant host cell comprising the nucleotide sequence of claim 28 or 29, or the vector of claim 30.

32. A hybridoma cell producing the anti-PD-L1 antibody or antigen binding fragment thereof of any one of claims 1-25.

33. A conjugate comprising the anti-PD-L1 antibody or antigen binding fragment thereof of any one of claims 1-25 or the bispecific antibody of claim 26 or 27, and a moiety conjugated thereto, wherein the moiety is selected from cytotoxins, radioisotopes, fluorescent labels, luminescent substances, chromogenic substances or enzymes.

34. A composition comprising the anti-PD-L1 antibody or antigen binding fragment thereof of any one of claims 1-25, the bispecific antibody of claim 26 or 27, or the conjugate of claim 33, and optionally one or more pharmaceutically acceptable carriers, excipients and/or diluents.

35. A method of treating cancer in a subject comprising administering to the subject the anti-PD-L1 antibody or antigen binding fragment thereof of any one of claims 1-25, the bispecific antibody of claim 26 or 27, the conjugate of claim 33, or the composition of claim 34.

36. The method of claim 35, wherein the cancer is selected from melanoma, lymphoma, bladder cancer, non-small cell lung cancer, head and neck cancer, colon cancer or skin cancer.

37. The method of claim 35 or 36, further comprising administering one or more additional therapeutic agents to the subject.

38. The method of claim 37, wherein the therapeutic agent is selected from an antibody, a chemotherapeutic drug, or a small molecule compound.

39. The method of claim 37 or 38, wherein the therapeutic agent targets an immune checkpoint molecule selected from PD-1, PD-L2, CTLA4, OX40, LAG3, TIM3, TIGIT, or CD103.

40. Use of the anti-PD-L1 antibody or antigen binding fragment thereof of any one of claims 1-25, the bispecific antibody of claim 26 or 27, the conjugate of claim 33, or the composition of claim 34 in the treatment of cancer in a subject.

41. Use of the anti-PD-L1 antibody or antigen binding fragment thereof of any one of claims 1-25, the bispecific antibody of claim 26 or 27, the conjugate of claim 33, or the composition of claim 34 in the manufacture of a medicament for treating cancer in a subject.

42. Use of claim 40 or 41, wherein the cancer is selected from melanoma, lymphoma, bladder cancer, non-small cell lung cancer, head and neck cancer, colon cancer or skin cancer.

43. Use of any one of claims 40-42, wherein the anti-PD-L1 antibody or antigen binding fragment thereof, conjugate or composition is administered in combination with one or more additional therapeutic agents.

44. Use of claim 43, wherein the therapeutic agent is selected from an antibody, a chemotherapeutic drug, or a small molecule compound.

45. Use of claim 43 or 44, wherein the therapeutic agent targets an immune checkpoint molecule selected from PD-1, PD-L2, CTLA4, OX40, LAG3, TIM3, TIGIT, or CD103.
